# EUROPEAN PATENT APPLICATION

(11) **EP 4 523 748 A2**
(43) Date of publication of application: **19.03.2025**
(21) Application number: 24210369.5
(22) Date of filing: 05.03.2021
(51) Int. Cl.: A61P 9/08

(54) **TREATMENT OF PAIN AND VASOCONSTRICTION**

(30) Priority: 06.03.2020 US 202062986544 P; 21.04.2020 US 202063013468 P
(62) Divisional of application: 21715057.2
(71) Applicant: Aisa Pharma, Inc., Boston, Massachusetts 02108 (US)
(72) Inventor: STERNLICHT, Andrew, Massachusetts, 02108 (US)
(74) Representative: D Young & Co LLP

(57) **Abstract**

Disclosed herein are methods of treating diseases and disorders using (i) dual N-type and L-type calcium channel blockers selective for the N-type calcium channel and/or (ii) Nav 1.7 sodium channel blockers, including cilnidipine.

## Description

### CROSS-REFERENCE TO RELATED APPLICATION

This application claims priority to U.S. Provisional Application No. 62/986,544, filed on March 6, 2020; and U.S. Provisional Application No. 63/013,468, filed on April 21, 2020, which are incorporated by reference herein in their entirety.

### TECHNICAL FIELD

This disclosure relates to methods of diseases and disorders using (i) dual N-type and L-type calcium channel blockers selective for the N-type calcium channel and/or (ii) Nav 1.7 sodium channel blockers, including cilnidipine.

### BACKGROUND

Neuropathic pain can be caused by diseases and disorders affecting the somatosensory system. Neuropathic pain can be a debilitating symptom and consequence of a variety of ailments including diabetes, amputation, various malignant or benign cancers, infectious diseases, hereditary conditions, nerve damage from any cause, amongst others. Neuropathic pain can be associated or present with abnormal sensations such as allodynia (pain from non-painful stimuli), hyperesthesia (increased sensation to stimuli), or dysesthesia (abnormal sensation commonly perceived as either painful, itchy, burning, or restrictive), and may have continuous and/or episodic (e.g., paroxysmal) components.

In certain diseases and disorders, neuropathic pain accompanies dysregulation of blood flow (e.g., vasoconstriction). For example, Raynaud's syndrome is a medical condition that is characterized by episodic and periodic reductions of blood flow (referred to as "attacks") to, e.g., the extremities, causing pain, numbness, discoloration, burning sensation, and neuropathic pain. Discoloration of the skin whose blood supply is reduced and then reperfused can accompany these symptoms. Tissue ischemia from reduced blood flow, as well as reperfusion when vasoconstriction ceases, produces, painful burning sensations which can be experienced by the subject during the ischemic attack as well as when blood flow is reestablished. Symptoms of Raynaud's syndrome can be experienced after, e.g., changes in temperature (cold or hot) in body tissues and/or the experience of strong emotions (e.g., stress) by the subject. In some subjects who are severely affected, symptoms can progress to digital ulceration and/or gangrene with limb or digit loss.

Existing treatments of Raynaud's syndrome and/or treatment of neuropathic pain can include surgical treatment (sympathectomy), although rare, to decrease sympathetic activity in the affected limb(s). More common methods of treatment include administration of calcium channel blockers for their vasodilating effect, which are recommended as first line pharmacotherapy, and avoidance of triggering exposures (e.g., changes in temperature or emotional events). However, side effects of calcium channel blockers commonly occur with use and can include, e.g., constipation, nausea, headache, fatigue, rash, edema, pulmonary edema, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, and itching. In some cases, administration of some types of calcium channel blockers can cause orthostatic hypotension. Other pharmacological approaches, including the use of phosphodiesterase inhibitors, prostanoids, angiotensin receptor blockers, endothelin receptor antagonists and topical nitroglycerin-containing creams have had marginal impact and often are accompanied by treatment-limiting side effects. These are considered second line treatment options, but a significant medical need still exists for an effective and safe pharmacological option for treatment for Raynaud's syndrome and the diseases in which it is included in the symptom complex of that disease.

### SUMMARY

Described herein are methods and compositions that include the use of dual N-type and L-type calcium channel blockers selective for the N-type calcium channel that can also, in some embodiments, possess sodium channel blocker activity (e.g., Nav 1.7 sodium channel blocker activity) for the treatment of diseases and disorders associated with sympathetic dysregulation (e.g., dysregulation of blood flow and sympathetic nervous system overactivity) in a subject. In some embodiments, the diseases and disorders are characterized by neuropathic pain and/or vasoconstriction.

N-type calcium channels are localized, e.g., at the sympathetic pre-synaptic nerve terminals and play a role in the release of neurotransmitters such as gamma-aminobutyric acid (GABA), acetylcholine, dopamine, and norepinephrine. N-type calcium channels are known to regulate, e.g., neuronal excitability and the firing of action potentials in the neurons, which increases the transmission of neurotransmitters in nociceptive pathways. These neurotransmitters then bind to the receptors on the sensory neurons that cause a person to feel pain. The induction of neuropathic pain can, in certain cases, be a result of the redistribution and alteration of subunit compositions of sodium and calcium channels that can result in spontaneous firing at abnormal locations along the sensory pathway. This may result in unpleasant sensory perceptions including, for example, burning pain, a feeling of wetness, itching, electrical shock pain, and the sensation of pins and needles.

Neuropathic pain is notoriously difficult to treat, with only 40-60% of patients achieving a degree of relief after treatment. Existing drugs have the potential for addiction and/or can cause serious side effects that are, without wishing to be bound by theory, believed to be at least in part theresult of unselective (e.g., non-discriminate or low selectivity) calcium channel inhibition.

Based on these considerations, N-selective dual N- and L-type calcium channel inhibition can be useful to treat diseases and disorders that are associated with dysregulation of blood flow and sympathetic nervous system overactivity, including those featuring symptoms of neuropathic pain.

A beneficial effect of L-type calcium channel inhibition is the dilation of the arteries in smooth muscle, causing an increase in arterial diameter, referred to as vasodilation. However, L-type calcium channel inhibition induces a homeostatic reflex mechanism in which norepinephrine is produced. The norepinephrine induces vasoconstriction, thus partially offsetting the vasodilating effects of the L-type calcium channel inhibition. A useful complementary effect of N-type calcium channel inhibition is the decrease of norepinephrine release and sympathetic outflow pre-synaptically in the spinal cord at the level of the dorsal root ganglion, which can counteract the homeostasis mechanism triggered by blockade of the L-type calcium channel. Disclosed herein are dual N-type and L-type calcium channel blockers selective for the N-type calcium channel (e.g., about 5-fold to 50-fold to about 100-fold selective) which can, for example, (1) reduce neuropathic pain, (2) induce vasodilation, and (3) counter the homeostatic vasoconstriction triggered by blockade of the L-type calcium channel. Dual N-type and L-type calcium channel blockers selective for the N-type calcium channel are therefore particularly effective at treating, e.g., Raynaud's disease.

Selective inhibition of the N-type calcium channel is, without wishing to be bound by theory, believed to result in reduced severity and/or frequency of side effects and increased tolerability compared to non-N-selective calcium channel blockade. Further, selective N-type CCBs may be effective for certain conditions at lower dosages and may provide higher efficacy relative to less selective calcium channel blockers. Additional advantages include an increase in bone density in certain subjects (e.g., subjects afflicted with osteoporosis) and beneficial renal effects. The beneficial renal effects are, without wishing to be bound by theory, believed to be an effect of reduced renal constriction, improvement in renal podocyte functioning, and improved blood flow in the kidney.

Additional advantages of selective N-channel blockade by CCBs, compared to CCBs that lack N-channel selectivity, can include:
- Improvement in endothelial function and endothelial concentrations of nitric oxide by improving blood flow, reducing pain that is, e.g., a consequence of reduced blood flow.
- Improvement in cardiac and left ventricle functioning resulting in reduction of pain due, e.g., to ischemia.
- Improvement in the incidence and severity of atherosclerosis including reducing pain caused, e.g., by a reduction in blood flow, and reducing the overall incidence of atherosclerotic-related events.
- Decrease in overall sympathetic nervous system activity and plasma concentration of norepinephrine, which can decrease pain due to net arteriole dilation and decrease in sympathetically mediated pain syndromes.
- Improvement in overall autonomic functioning, which may improve gut function in patients whose gut function (e.g., competency of the lower esophageal sphincter and peristalsis and gastric emptying), is compromised due to impaired autonomic function as occurs in certain disease states (e.g., scleroderma).

Selective N-type CCBs are also amenable to combination with other agents which may have an additive or complementary effect with the selective N-type CCB. For example, selective N-type CCBs may reduce the blood pressure of a hypertensive subject, which can be counterbalanced by combining the selective N-type CCB with an agent that increases blood pressure. Without wishing to be bound by theory, the selective N-type CCBs do not lower the blood pressure of a normotensive subject (i.e., a subject that does not have abnormal blood pressure; e.g., a subject that does not have hypertension). Further, the vasodilating effects of selective N-type CCBs can improve the effectiveness of an agent that treats erectile dysfunction. In addition, the vasodilating effects of selective N-type CCBs can improve the effectiveness of other agents used as second line treatment for patients having, e.g., Raynauds disease and scleroderma. Cilnidipine exerts a balance of selective N- vs. L-type calcium channel inhibition (which can have a 5 fold to 50-fold to 100-fold selectivity for N-type calcium channel over L-type calcium channel), making it surprisingly effective at treating diseases and disorders characterized by neuropathic pain and vasoconstriction.

In one aspect, disclosed herein is a method of treating a disease or disorder associated with dysregulation of blood flow and sympathetic nervous system overactivity in a subject, comprising administering a therapeutically effective amount of a dual N-type and L-type calcium channel blocker selective for the N-type calcium channel to the subject.

In another aspect, disclosed herein is a method of treating a disease or disorder characterized by vasoconstriction or neuropathic pain in a subject in need thereof, the method comprising (a) determining that the disease or disorder is associated with vasoconstriction or neuropathic pain; and (b) administering to the subject a therapeutically effective amount of a dual N-type and L-type calcium channel blocker selective for the N-type calcium channel.

In another aspect, disclosed herein is a method of reducing a sensation of burning pain, paresthesia, dysesthesia, hypoesthesia, allodynia, or hyperesthesia in a subject in need thereof, comprising administering a therapeutically effective amount of a dual N-type and L-type calcium channel blocker selective for the N-type calcium channel to a subject.

In another aspect, disclosed herein is a method of reducing pain or discomfort in a subject in need thereof caused by a reduction of body temperature in the subject, comprising administering an effective amount of a dual N-type and L-type calcium channel blocker selective for the N-type calcium channel to the subject, wherein the reduction of body temperature in the subject is caused by an exposure of the subject to air having a temperature of less than 25°C.

In another aspect, disclosed herein is a method of reducing susceptibility of a subject to cold-induced pain or discomfort, comprising: administering an effective amount of a dual N-type and L-type calcium channel blocker selective for the N-type calcium channel to the subject.

In another aspect, disclosed herein is a method of treating cold-induced pain or discomfort, comprising: administering an effective amount of a dual N-type and L-type calcium channel blocker selective for the N-type calcium channel to the subject.

In another aspect, disclosed herein is a method of treating sickle cell disease in a subject in need thereof, comprising administering an effective amount of a dual N-type and L-type calcium channel blocker selective for the N-type calcium channel to the subj ect.

In another aspect, disclosed herein is a method of treating vasculitis in a subject in need thereof, comprising administering an effective amount of a dual N-type and L-type calcium channel blocker selective for the N-type calcium channel to the subject.

In another aspect, disclosed herein is a method of treating thrombosis in a subject in need thereof, comprising administering an effective amount of a dual N-type and L-type calcium channel blocker selective for the N-type calcium channel to the subject.

In another aspect, disclosed herein is a method of treating a kidney disorder in a subject in need thereof, comprising administering an effective amount of a dual N-type and L-type calcium channel blocker selective for the N-type calcium channel to the subject, wherein the kidney disorder is a complication of a disease or disorder.

In another aspect, disclosed herein is a method of treating hypertension and chronic kidney disease in a subject in need thereof, comprising administering an effective amount of a dual N-type and L-type calcium channel blocker selective for the N-type calcium channel to the subject.

In another aspect, disclosed herein is a method of relieving eye pain in a subject in need thereof, comprising administering a solution of a therapeutically effective amount of a dual N-type and L-type calcium channel blocker selective for the N-type calcium channel to the eye of the subject.

In another aspect, disclosed herein is a method of treating atrial fibrillation in a subject in need thereof, comprising administering a dual N-type and L-type calcium channel blocker selective for the N-type calcium channel to the subject, wherein after the administration the atrial fibrillation in the subject is improved.

In another aspect, disclosed herein is a method of reducing atrial remodeling in a subject with atrial fibrillation, comprising: administering a dual N-type and L-type calcium channel blocker selective for the N-type calcium channel to the subject.

In another aspect, disclosed herein is a method of improving blood flow in a subject after surgery or revascularization in the subject, comprising administering a dual N-type and L-type calcium channel blocker selective for the N-type calcium channel to the subject. Improvement in blood flow can occur either through net vasodilation effects or the prevention/treatment of vasospasm in the subject.

In another aspect, disclosed herein is a method of improving blood flow in a subject exhibiting reduced blood flow, comprising administering a dual N-type and L-type calcium channel blocker selective for the N-type calcium channel to the subject, wherein after administration blood flow in the subject is improved.

In another aspect, disclosed herein is a method of treating erectile dysfunction in a subject in need thereof, comprising administering a dual N-type and L-type calcium channel blocker selective for the N-type calcium channel and an agent that treats erectile dysfunction to the subject, wherein after administration erectile dysfunction in the subject is improved.

In another aspect, disclosed herein is a method of treating a disease or disorder associated with dysregulation of blood flow and sympathetic nervous system overactivity in a subject in need thereof, comprising administering a therapeutically effective amount of a Nav 1.7 sodium channel blocker to the subject.

In another aspect, disclosed herein is a method of treating a disease or disorder associated with dysregulation of blood flow and sympathetic nervous system overactivity in a subject in need thereof, comprising administering a therapeutically effective amount of a Nav 1.7 sodium channel blocker to the subject.

In another aspect, disclosed herein is a pharmaceutical composition comprising a dual N-type and L-type calcium channel blocker selective for the N-type calcium channel, an agent that increases blood pressure, and optionally a pharmaceutically acceptable excipient.

In another aspect, disclosed herein is a pharmaceutical composition comprising a dual N-type and L-type calcium channel blocker selective for the N-type calcium channel, an agent that treats erectile dysfunction, and optionally a pharmaceutically acceptable excipient.

In another aspect, disclosed herein is a pharmaceutical composition comprising a dual N-type and L-type calcium channel blocker selective for the N-type calcium channel, a calcineurin inhibitor, and optionally a pharmaceutically acceptable excipient.

### Definitions

As used herein, the terms "about" and "approximately" are used interchangeably, and when used to refer to modify a numerical value, encompass a range of uncertainty of the numerical value of from 0% to 10% of the numerical value.

As used herein, the singular forms "a," "an," and "the" include plural referents unless the context clearly dictates otherwise.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment.

As used herein, the terms "subject" "individual," or "patient," are used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the disease or disorder is associated with dysregulation of blood flow and sympathetic nervous system overactivity. In some embodiments, the disease or disorder is characterized by neuropathic pain, vasoconstriction, dysesthetic pain, burning pain, body temperature changes of the subject, hyperesthesia, changes in skin or tissue color, edema, changes in skin turgor, ruble, pallor, cyanosis, vasospasm, or any combination thereof.

As used herein, the phrase "dialysis support" refers to the assistance in maintaining concentrations of solutes (e.g., urea and/or creatinine) in a subject's blood within ranges that occur in subjects that have normal kidney function.

As used herein, the phrase "fixed dosage form" refers to the simultaneous administration of a dual N-type and L-type calcium channel blocker selective for the N-type calcium channel and at least one additional therapeutic agent (e.g., a calcineurin inhibitor, a non-steroidal anti-inflammatory drug, or both to a subject in the form of a single composition or dosage.

As used herein, the phrase "dysregulation of blood flow and sympathetic nervous system overactivity" refers to reduced blood flow in any part of a subject's body and concomitant increased sympathetic outflow in the brainstem of the subject. In some embodiments, increased sympathetic outflow is evidenced by a decrease in heart function. In some embodiments, cardiac autonomic nervous function and thermographic (e.g., infrared thermographic) parameters can be assessed to indicate whether a subject exhibits dysregulation of blood flow and/or sympathetic nervous system overactivity. In certain embodiments, assessment of cardiac autonomic nervous function and thermographic parameters enables, e.g., a comparison of the function of the cardiac autonomic nervous system and the peripheral response to cold exposure. See clinical trial NCT 03094910 at clinicaltrials.gov/ct2/show/NCT03094910, which is incorporated by reference herein in its entirety. In some embodiments, sympathetic nervous system overactivity can be assessed before and/or during a standardized mental arithmetic test. In certain embodiments, the mental arithmetic test can be conducted in a climate room. Parameters that can be measured during the mental arithmetic test include, but are not limited to, blood pressure, heart rate, forearm blood flow, fingertip laser Doppler flux, and/or venous concentrations of norepinephrine and epinephrine from the back of the hand. In some embodiments, measurement(s) of the foregoing parameters are compared to a control group that is not afflicted with dysregulation of blood flow and/or sympathetic nervous system overactivity. In some embodiments, indications of sympathetic nervous system overactivity include, but are not limited to, an increase in laser Doppler flux relative to the control group, a higher baseline diastolic blood pressure relative to the control group, a higher heart rate relative to the control group, a higher venous concentration of epinephrine relative to the control group, and/or higher a venous concentration of norepinephrine relative to the control group. See Int. Angiol., 1990, 9(2), 84-89, which is incorporated by reference herein in its entirety.

As used herein, the term "dual N-type and L-type calcium channel blocker selective for the N-type calcium channel", "selective N-type calcium channel blocker", "selective N-type CCB", and "N-type selective CCB" refer to an agent that inhibits both N- and L-type calcium channels, and inhibits the N-type calcium channel to a greater degree than the L-type calcium channel. In some embodiments, the dual N-type and L-type calcium channel blocker selective for the N-type calcium channel has at least a 5-fold selectivity for the N-type calcium channel over the L-type calcium channel. For example, the dual N-type and L-type calcium channel blocker selective for the N-type calcium channel exhibits at least a 10-fold, at least a 30-fold, at least a 50-fold, at least a 80-fold, at least a 100-fold, at least a 300-fold, at least a 500-fold, at least a 800-fold, at least a 900-fold, or at least a 1000-fold selectivity for the N-type calcium channel over the L-type calcium channel. For example, the dual N-type and L-type calcium channel blocker selective for the N-type calcium channel exhibits at least a 50-fold to 100-fold selectivity for the N-type calcium channel over the L-type calcium channel. Examples of dual N-type and L-type calcium channel blocker selective for the N-type calcium channel include, but are not limited to, cilnidipine, Z-160, ralfinamide, CNV2197944, or pharmaceutically acceptable salts thereof. Examples of dual N-type and L-type calcium channel blocker selective for the N-type calcium channel include, but are not limited to, cilnidipine, Z-160, CNV2197944, or pharmaceutically acceptable salts thereof. Examples of dual N-type and L-type calcium channel blocker selective for the N-type calcium channel include, but are not limited to, cilnidipine, Z-160, zicinotide, and pharmaceutically acceptable salts thereof.

In some embodiments, the dual N-type and L-type calcium channel blocker selective for the N-type calcium channel is also a Nav 1.7 sodium channel blocker (i.e., the dual N-type and L-type calcium channel blocker selective for the N-type calcium channel further inhibits a Nav 1.7 sodium channel). In some embodiments, the dual N-type and L-type calcium channel blocker selective for the N-type calcium channel that further inhibits a sodium channel (e.g., Nav 1.7) is cilnidipine. As used herein, the term "non-N-type selective calcium channel blocker" refers to an agent that blocks one or more calcium channels, but either (1) does not block the N-type calcium channel, or (2) blocks the N-type calcium channel, but not selectively over the L-type calcium channel. Examples of non-N-type selective calcium channel blockers include, but are not limited to, nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine and nitrendipine, and pharmaceutical salts thereof.

As used herein, the term "Nav 1.7 sodium channel blocker" or "Nav 1.7 sodium channel inhibitor" refers to an agent that can inhibit the Nav 1.7 sodium channel. In some embodiments, the Nav 1.7 sodium channel blocker inhibits the closed state of the Nav 1.7 sodium channel. In some embodiments, the Nav 1.7 sodium channel blocker inhibits the inactivated state of the Nav 1.7 sodium channel. In some embodiments, the Nav 1.7 sodium channel blocker is also a dual N-type and L-type calcium channel blocker selective for the N-type calcium channel (i.e., the Nav 1.7 sodium channel blocker further inhibits the N-type and L-type calcium channels selectively for the N-type calcium channel). In some embodiments, the Nav 1.7 sodium channel blocker that further inhibits the N-type and L-type calcium channels selectively for the N-type calcium channel is cilnidipine.

As used herein, the term "adverse effect" refers to an undesirable effect resulting from an alteration in normal physiology in a subject.

As used herein, the phrase "analgesia of the eye" refers to pain relief of the eye.

As used herein, the phrase "anesthesia of the eye" refers to numbing of the eye.

As used herein, the term "vasoconstriction" refers to the reduction in diameter of a blood vessel (e.g., an artery, vein, or capillary) resulting in reduced blood flow to the tissue the vasoconstricted blood vessels circulate blood to and from.

As used herein, the term "reducing susceptibility of a subject to cold-induced pain or discomfort" refers to reducing the pathologic response of a subject to experience pain or discomfort when subjected to an environment that lowers the temperature of a body part of the subject. In some embodiments, reducing susceptibility of a subject to cold-induced pain or discomfort can include reducing the likelihood that a subject will experience pain or discomfort when subjected to an environment that lowers the temperature of a body part of the subject. In some embodiments, reducing susceptibility of a subject to cold-induced pain or discomfort can include reducing the magnitude or intensity of pain or discomfort that a subject feels when subjected to an environment that lowers the temperature of a body part of the subject.

As used herein, the term "body temperature" refers to the temperature range of the body in a healthy, awake subject under normal conditions of thermoregulation as measured in the mouth, the rectum, the armpit, or the ear. For example, the temperature range in a healthy human subject under normal conditions of thermoregulation is 36.1 °C to 37.8 °C.

The term "therapeutically effective amount," as used herein, refers to a sufficient amount of a chemical entity (e.g., a dual N-type and L-type calcium channel blocker selective for the N-type calcium channel and/or a Nav 1.7 sodium channel blocker) being administered which will relieve to an extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The term "pharmaceutically acceptable excipient" means a pharmaceutically-acceptable material, composition, or vehicle, such as a liquid or solid filler, diluent, carrier, solvent, or encapsulating material. In one embodiment, each component is " pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutically acceptable salt" may refer to pharmaceutically acceptable addition salts prepared from pharmaceutically acceptable non-toxic acids including inorganic and organic acids. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. The term "pharmaceutically acceptable salt" may also refer to pharmaceutically acceptable addition salts prepared by reacting a compound having an acidic group with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and salts with amino acids such as arginine, lysine, and the like, or by other methods previously determined. The pharmacologically acceptable salt s not specifically limited as far as it can be used in medicaments. Examples of a salt that the compounds described hereinform with a base include the following: salts thereof with inorganic bases such as sodium, potassium, magnesium, calcium, and aluminum; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid:organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

The term "pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: transdermal, intranasally, sublingual, intraspinal, or ocular administration.

The details of one or more embodiments of the invention are set forth in the description below. Other features and advantages of the invention will be apparent from the description and drawings, and from the claims.

### BRIEF DESCRIPTION OF THE DRAWINGS

FIG. 1 depicts a schematic diagram based on the modulated hypothesis of drug binding to various states of a sodium channel (NaCh).
FIG. 2 depicts a two pulse voltage protocol. The line marked with an asterisk indicates the voltage that was applied to the cell.
FIG. 3A is an overlay of inactivation current traces from VP1.
FIG. 3B depicts current traces at closed and inactivated states from the two pulse voltage protocol (VP2).
FIG. 4A depicts an It-plot for amlodipine at the closed state (P1).
FIG. 4B depicts an It-plot for amlodipine at the inactivated state (P2).
FIG. 5A depicts an It-plot for cilnidipine at the closed state (P1).
FIG. 5B is an It-plot for cilnidipine at the inactivated state (P2).
FIG. 6A depicts an It-plot for gabapentin at the closed state (P1).
FIG. 6B depicts an It-plot for gabapentin at the inactivated state (P2).
FIG. 7A depicts an example of an It-plot for bupivacaine at the closed state (P1).
FIG. 7B depicts an It-plot for bupivacaine at the inactivated state (P2).
FIG. 8A depicts a group hill fit for amlodipine at the closed state (P1).
FIG. 8B depicts a group hill fit for amlodipine at the inactivated state (P2).
FIG. 9A depicts a group hill fit for cilnidipine at the closed state (P1).
FIG. 9B depicts a group hill fit for cilnidipine at the inactivated state (P2).
FIG. 10A depicts a group hill fit for gabapentin at the closed state (P1).
FIG. 10B depicts a group hill fit for gabapentin at the (P2) inactivated state.
FIG. 11A depicts a group hill fit for bupivacaine at closed state (P1).
FIG. 11B depicts a group hill fit for bupivacaine at (P2) inactivated state.
FIG. 12 depicts percent Nav 1.7 sodium channel inhibition at Cₘₐₓ for nifedipine 40 mg, cilnidipine 10 mg, cilnidipine 20 mg, amlodipine 10 mg, gabapentin 1800 mg, and bupivacaine 1 µM.

### DETAILED DESCRIPTION

In one aspect, disclosed herein is a method of treating a disease or disorder associated with dysregulation of blood flow and sympathetic nervous system overactivity in a subject in need thereof, comprising administering a therapeutically effective amount of a dual N-type and L-type calcium channel blocker selective for the N-type calcium channel to the subject. In some embodiments, the disease or disorder associated with dysregulation of blood flow and sympathetic nervous system overactivity is a disease or disorder characterized by vasoconstriction or neuropathic pain.

Examples of N-type calcium channels include, but are not limited to, the Cav2.2 Type, which has two subunits, Cav 2.2a and Cav2.2b, both of which have an alpha 1 subunit of 2.2 and are affected by N type current.

In some embodiments, the therapeutically effective amount of the dual N-type and L-type calcium channel blocker selective for the N-type calcium channel is reduced compared to the therapeutically effective amount of a non-N-selective calcium channel blocker useful to treat the disease or disorder.

In some embodiments, one or more side effects experienced by the subject after administration of the dual N-type and L-type calcium channel blocker selective for the N-type calcium channel are less severe or less frequent than as compared to the side effects experienced by a subject after administration of a therapeutically effective amount of a non-N-selective calcium channel blocker useful to treat the disease or disorder. Without wishing to be bound by theory, this may allow a higher dose of the dual N-type and L-type calcium channel blocker selective for the N-type calcium channel to be administered to the subject, which can, e.g., result in a higher treatment efficacy than the non-N-selective calcium channel blocker.

In some embodiments, the dual N-type and L-type calcium channel blocker selective for the N-type calcium channel is more effective than the non-N-selective calcium channel blocker in treating an adverse effect of the disease or disorder. In some embodiments, the adverse effect is a symptom or a clinical manifestation of the disease or disorder.

It has been shown that dual N-type and L-type calcium channel blockers selective for the N-type calcium channels have fewer and less severe side effects, better tolerability, and are safer than less-N-selective calcium channel blockers. It is believed that this is due to the increased inhibition of the N channel relative to the L channel. By decreasing sympathetic activity, as well as by dilating not only arterioles but the venous system, dual N-type and L-type calcium channel blockers selective for the N-type calcium channel appear to be associated with less adverse events in patients treated for hypertension than patients treated with dual L and N- calcium channel antagonists with lower levels of N - selectivity.

In some embodiments, the disease or disorder associated with dysregulation of blood flow and sympathetic nervous system overactivity is characterized by neuropathic pain (e.g., chronic neuropathic pain), vasoconstriction, dysesthetic pain, burning pain, hyperesthetic pain, allodynia, lancinating pain, crampy pain, dull pain, body temperature changes of the subject, hyperesthesia, changes in skin or tissue color, edema, changes in skin turgor, rubor, pallor, cyanosis, vasospasm, or any combination thereof. In certain embodiments, the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia, or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence. In certain of these embodiments, the disease or disorder is Raynaud's syndrome. For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In some embodiments, the non-N-selective calcium channel blocker is a dihydropyridine. In some other embodiments, the non-N-selective calcium channel blocker is a non-dihydropyridine. Non-limiting examples of non-N-selective calcium channel blockers include, but are not limited to: nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine and nitrendipine, and pharmaceutically acceptable salts thereof.

In some embodiments, the therapeutically effective amount of the dual N-type and L-type calcium channel blocker selective for the N-type calcium channel is at least 10% lower than the therapeutically effective amount of the non-N-selective calcium channel blocker. For example, the therapeutically effective amount of the dual N-type and L-type calcium channel blocker selective for the N-type calcium channel is at least 15% lower, at least 20% lower, at least 25% lower, at least 30% lower, at least 35% lower, at least 40% lower, at least 45% lower, at least 50% lower, at least 55% lower, at least 60% lower, at least 65% lower, at least 70% lower, at least 75% lower, at least 80% lower, at least 85% lower, at least 90% lower, or at least 95% lower than the therapeutically effective amount of the non-N-selective calcium channel blocker.

In some embodiments, the therapeutically effective amount of the dual N-type and L-type calcium channel blocker selective for the N-type calcium channel decreases the blood pressure (e.g., the systolic blood pressure) of the subject to a lesser degree than the therapeutically effective amount of the non-N-selective calcium channel blocker. In some embodiments, the dual N-type and L-type calcium channel blocker selective for the N-type calcium channel decreases the blood pressure (e.g., the systolic blood pressure) of the subject at least 5% less than the non-N-selective calcium channel blocker. For example, the dual N-type and L-type calcium channel blocker selective for the N-type calcium channel decreases the blood pressure (e.g., the systolic blood pressure) of the subject at least 10% less, at least 15% less, at least 20% less, at least 25% less, at least 30% less, at least 35% less, at least 40% less, at least 45% less, at least 50% less, at least 55% less, at least 60% less, at least 65% less, at least 70% less, at least 75% less, at least 80% less, at least 85% less, at least 90% less, or at least 95% less, than the non-N-selective calcium channel blocker.

In some embodiments, the side effects are selected from the group consisting of: constipation, nausea, headache, fatigue, rash, edema, pulmonary edema, peripheral edema, heart rate changes, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, and itching.

In some embodiments, the subject is also diagnosed with hypertension; and wherein after administration of the dual N-type and L-type calcium channel blocker selective for the N-type calcium channel to the subject, the blood pressure (e.g., the systolic blood pressure) of the subject is reduced.

In some embodiments, the subject has hypertension; and wherein after administration of the dual N-type and L-type calcium channel blocker selective for the N-type calcium channel to the subject, the blood pressure (e.g., the systolic blood pressure) of the subject is reduced.

In some embodiments, the subject is being treated for hypertension; and wherein after administration of the dual N-type and L-type calcium channel blocker selective for the N-type calcium channel to the subject, the blood pressure (e.g., the systolic blood pressure) of the subject is reduced.

In some embodiments, the subject also has hypertension; and wherein after administration of the dual N-type and L-type calcium channel blocker selective for the N-type calcium channel to the subject, the blood pressure (e.g., the systolic blood pressure) of the subject is reduced. In some embodiments, the subject does not have hypertension; and wherein after administration of the dual N-type and L-type calcium channel blocker selective for the N-type calcium channel to the subject, the blood pressure (e.g., the systolic blood pressure) of the subject is not reduced. Without wishing to be bound by theory, it is believed that when the subject has hypertension, the dual N-type and L-type calcium channel blocker selective for the N-type calcium channel reduces the blood pressure of the subject; however, when the subject does not have hypertension (i.e., the subject is normotensive), the dual N-type and L-type calcium channel blocker selective for the N-type calcium channel does not reduce the blood pressure of the subject. In some embodiments, after administration of the dual N-type and L-type calcium channel blocker selective for the N-type calcium channel to the subject, the cardiac function of the subject is improved (e.g., left ventricular function of the subject is improved).

In some embodiments, after administration of the dual N-type and L-type calcium channel blocker selective for the N-type calcium channel to the subject, the cardiac function of the subject is improved. In some embodiments, improving the cardiac function in the subject comprises improving the left ventricular function of the subject. In some embodiments, the subject has hypertension. In some embodiments, the subject does not have hypertension.

In some embodiments, the subject has hypertension; and after administration of the dual N-type and L-type calcium channel blocker selective for the N-type calcium channel to the subject, the blood pressure of the subject is decreased and the cardiac function (e.g., left ventricular function) of the subject is improved.

In some embodiments, the subject does not have hypertension; and after administration of the dual N-type and L-type calcium channel blocker selective for the N-type calcium channel to the subject, the blood pressure of the subject is not decreased and the cardiac function (e.g., left ventricular function) of the subject is improved.

In some embodiments, the subject has hypertension and osteoporosis; and after administration of the dual N-type and L-type calcium channel blocker selective for the N-type calcium channel to the subject, the blood pressure of the subject is decreased and the bone density in the subject is increased.

In some embodiments, the subject does not have hypertension; the subject has osteoporosis; and after administration of the dual N-type and L-type calcium channel blocker selective for the N-type calcium channel to the subject, the blood pressure of the subject is not decreased and the bone density in the subject is increased.

In some embodiments, the subject has hypertension; and after administration of the dual N-type and L-type calcium channel blocker selective for the N-type calcium channel to the subject, the blood pressure of the subject is decreased and the bone density in the subject is increased.

In some embodiments, the subject does not have hypertension; and after administration of the dual N-type and L-type calcium channel blocker selective for the N-type calcium channel to the subject, the blood pressure of the subject is not decreased and the bone density in the subject is increased.

In some embodiments, the subject has hypertension and atherosclerosis; and after administration of the dual N-type and L-type calcium channel blocker selective for the N-type calcium channel to the subject, the blood pressure of the subject is decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject does not have hypertension; the subject has atherosclerosis; and after administration of the dual N-type and L-type calcium channel blocker selective for the N-type calcium channel to the subject, the blood pressure of the subject is not decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has hypertension; and after administration of the dual N-type and L-type calcium channel blocker selective for the N-type calcium channel to the subject, the blood pressure of the subject is decreased and renal function in the subject is improved.

In some embodiments, the subject does not have hypertension; and after administration of the dual N-type and L-type calcium channel blocker selective for the N-type calcium channel to the subject, the blood pressure of the subject is not decreased and renal function in the subject is improved.

In some embodiments, the subject has hypertension; and after administration of the dual N-type and L-type calcium channel blocker selective for the N-type calcium channel to the subject, the blood pressure of the subject is decreased and renal function in the subject is improved. In some embodiments, the subject does not have hypertension; and after administration of the dual N-type and L-type calcium channel blocker selective for the N-type calcium channel to the subject, the blood pressure of the subject is not decreased and renal function in the subject is improved.

In some embodiments, the subject has hypertension; the subject was previously treated with antihypertensive agents before administration of the dual N-type and L-type calcium channel blocker selective for the N-type calcium channel; and after administration of the dual N-type and L-type calcium channel blocker selective for the N-type calcium channel to the subject, the blood pressure of the subject is decreased.

In some embodiments, the subject does not have hypertension; the subject was previously treated with antihypertensive agents before administration of the dual N-type and L-type calcium channel blocker selective for the N-type calcium channel; and after administration of the dual N-type and L-type calcium channel blocker selective for the N-type calcium channel to the subject, the blood pressure of the subject is is not decreased.

In some embodiments, the subject has hypertension; the subject has scleroderma; the subject has a digital ulcer; and after administration of the dual N-type and L-type calcium channel blocker selective for the N-type calcium channel to the subject, the blood pressure of the subject is decreased, and the digital ulcer is treated. In some embodiments, treating the digital ulcer comprises healing or improving the condition of the digital ulcer.

In some embodiments, the subject does not have hypertension; the subject has scleroderma; the subject has a digital ulcer; and after administration of the dual N-type and L-type calcium channel blocker selective for the N-type calcium channel to the subject, the blood pressure of the subject is not reduced, and the digital ulcer is treated. In some embodiments, treating the digital ulcer comprises healing or improving the condition of the digital ulcer.

In some embodiments, the subject has hypertension; the subject has scleroderma; the disease or disorder associated with dysregulation of blood flow and sympathetic nervous system overactivity is Raynaud's syndrome; and after administration of the dual N-type and L-type calcium channel blocker selective for the N-type calcium channel to the subject, the blood pressure of the subject is decreased.

In some embodiments, the subject does not have hypertension; the subject has scleroderma; the disease or disorder associated with dysregulation of blood flow and sympathetic nervous system overactivity is Raynaud's syndrome; and after administration of the dual N-type and L-type calcium channel blocker selective for the N-type calcium channel to the subject, the blood pressure of the subject is not reduced.

In some embodiments, the blood pressure (e.g., systolic blood pressure) of the subject is reduced by greater than 1 mm Hg. For example, the systolic blood pressure of the subject is reduced by greater than 2, 5, 8, 10, 12, 15, 20, 25, or 30 mm Hg. For example, the systolic blood pressure of the subject is reduced by about 1-5 mm Hg, about 5-10 mm Hg, about 10-15 mm Hg, about 15-20 mm Hg, or about 20-30 mm Hg.

In some embodiments, the subject has atherosclerosis; and after administration of the dual N-type and L-type calcium channel blocker selective for the N-type calcium channel to the subject, the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has a digital ulcer; and after administration of the dual N-type and L-type calcium channel blocker selective for the N-type calcium channel to the subject, the digital ulcer is treated. In some embodiments, treating the digital ulcer comprises healing or improving the condition of the digital ulcer.

In some embodiments, the subject is also being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject has lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof. In some embodiments, the treatment for lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof comprises administering a therapeutic agent. Therapeutic agents known in the art for treating lupus, scleroderma, scleroderma with interstitial lung disease, rheumatoid arthritis, atherosclerosis, cryoglobulinemia, polycythemia, dermatomyositis, polymyositis, and Sjögren's syndrome can be found in, e.g., the Physicians' desk reference. (71st ed.). (2017). Montvale, NJ: PDR Network.

In certain embodiments, the subject is being treated for scleroderma. For example, the subject is being treated for scleroderma with interstitial lung disease. In certain embodiments, the subject has scleroderma. For example, the subject has scleroderma with interstitial lung disease. In certain embodiments, the subject is diagnosed with scleroderma. For example, the subject is diagnosed with scleroderma with interstitial lung disease. In some embodiments, the method further comprises administering an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the method further comprises administering nintedanib. In some embodiments, the method further comprises administering a calcineurin inhibitor, a non-steroidal anti-inflammatory drug, or both. In some embodiments, the dual N-type and L-type calcium channel blocker selective for the N-type calcium channel, the calcineurin inhibitor, and the non-steroidal anti-inflammatory drug are administered separately, sequentially, or simultaneously. In some embodiments, the dual N-type and L-type calcium channel blocker selective for the N-type calcium channel, the calcineurin inhibitor, and the non-steroidal anti-inflammatory drug are administered separately. In some embodiments, the dual N-type and L-type calcium channel blocker selective for the N-type calcium channel, the calcineurin inhibitor, and the non-steroidal anti-inflammatory drug are administered sequentially. In some embodiments, the dual N-type and L-type calcium channel blocker selective for the N-type calcium channel, the calcineurin inhibitor, and the non-steroidal anti-inflammatory drug are administered simultaneously. In some embodiments, the dual N-type and L-type calcium channel blocker selective for the N-type calcium channel, the calcineurin inhibitor, and the non-steroidal anti-inflammatory drug are administered simultaneously as a fixed dosage form. In some embodiments, the calcineurin inhibitor is a cyclosporine. In some embodiments, the non-steroidal anti-inflammatory drug is aspirin.

In certain embodiments, the subject is being treated for lupus. In certain embodiments, the subject is diagnosed with lupus. In certain embodiments, the subject has lupus. In some embodiments, the method further comprises administering an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the subject is being treated for rheumatoid arthritis. In certain embodiments, the subject has rheumatoid arthritis. In certain embodiments, the subject is diagnosed with rheumatoid arthritis. In some embodiments, the method further comprises administering an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the subject is being treated for Sjögren's syndrome. In certain embodiments, the subject has Sjögren's syndrome. In certain embodiments, the subject is diagnosed with Sjögren's syndrome. In some embodiments, the method further comprises administering an agent selected from the group consisting of: plaquenil, an antimalarial drug (e.g., hydroxychloroquine), evoxac, cevimeline, infliximab, or any combination thereof.

In certain embodiments, the subject is being treated for idiopathic pulmonary fibrosis. In certain embodiments, the subject has idiopathic pulmonary fibrosis. In certain embodiments, the subject is diagnosed with idiopathic pulmonary fibrosis. In some embodiments, the method further comprises administering an agent selected from the group consisting of: nintedanib, pirfenidone, or any combination thereof.

In some embodiments, the disease or disorder associated with dysregulation of blood flow and sympathetic nervous system overactivity in a subject is secondary Raynaud's disease; the subject is also being treated for scleroderma; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder associated with dysregulation of blood flow and sympathetic nervous system overactivity in a subject is secondary Raynaud's disease; the subject is also being treated for scleroderma with interstitial lung disease; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder associated with dysregulation of blood flow and sympathetic nervous system overactivity in a subject is secondary Raynaud's disease; the subject is also being treated for lupus; the dual N-type and L-type calcium channel blocker selective for the N-type calcium channel is cilnidipine; and the subject is further administered an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In some embodiments, the disease or disorder associated with dysregulation of blood flow and sympathetic nervous system overactivity in a subject is secondary Raynaud's disease; the subject is also being treated for rheumatoid arthritis; the dual N-type and L-type calcium channel blocker selective for the N-type calcium channel is cilnidipine; and the subject is further administered an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In some embodiments, the disease or disorder associated with dysregulation of blood flow and sympathetic nervous system overactivity in a subject is secondary Raynaud's disease; the subject is also being treated for Sjögren's syndrome; the dual N-type and L-type calcium channel blocker selective for the N-type calcium channel is cilnidipine; and the subject is further administered an agent selected from the group consisting of: plaquenil, an antimalarial drug (e.g., hydroxychloroquine), evoxac, cevimeline, infliximab, or any combination thereof.

In some embodiments, the disease or disorder associated with dysregulation of blood flow and sympathetic nervous system overactivity in a subject is secondary Raynaud's disease; the subject is also being treated for idiopathic pulmonary fibrosis; the dual N-type and L-type calcium channel blocker selective for the N-type calcium channel is cilnidipine; and the subject is further administered an agent selected from the group consisting of: nintedanib, pirfenidone, or any combination thereof.

In some embodiments, the adverse effect is pain (e.g., neuropathic pain or migraine (e.g., congenital migraine)), cerebellar ataxia, angina, epilepsy, hypertension, ischemia, and arrhythmia. In certain embodiments, the pain is neuropathic pain. For example, the neuropathic pain is burning pain, a feeling of wetness, pruritis (itching), electrical shock pain, the sensation of pins and needles, a lancinating pain, a dull pain, or a crampy pain. In certain embodiments the pain is a lancinating pain, a dull pain, or a crampy pain. In some embodiments, the adverse effect is numbness, sedation, decreased respiratory rate, constipation, disorientation, tachycardia, hyperkinetic movements, addiction (e.g., alcohol addiction), and inflammation.

In some embodiments, the adverse effect is vasoconstriction. In certain embodiments, the vasoconstriction comprises vasoconstriction of a body part, and the temperature of the vasoconstricted body part is lower than the subject's body temperature. In certain of these embodiments, the dual N-type and L-type calcium channel blocker selective for the N-type calcium channel increases the temperature of the vasoconstricted body part to the subject's body temperature at least 5% faster than the non-N-selective calcium channel blocker. For example, the dual N-type and L-type calcium channel blocker selective for the N-type calcium channel increases the temperature of the vasoconstricted body part to the subject's body temperature at least 10% faster, at least 15% faster, at least 20% faster, at least 25% faster, at least 30% faster, at least 35% faster, at least 40% faster, at least 45% faster, at least 50% faster, at least 55% faster, at least 60% faster, at least 65% faster, at least 70% faster, at least 75% faster, at least 80% faster, at least 85% faster, at least 90% faster, or at least 95% faster than the non-N-selective calcium channel blocker. In some embodiments, the difference can be measured in seconds or minutes.

In another aspect, disclosed herein is a method of treating a disease or disorder characterized by vasoconstriction or neuropathic pain in a subject in need thereof, the method comprising (a) determining that the disease or disorder is associated with dysregulation of blood flow and sympathetic nervous system overactivity; and (b) administering to the subject a therapeutically effective amount of a dual N-type and L-type calcium channel blocker selective for the N-type calcium channel.

In another aspect, disclosed herein is a method of treating a disease or disorder characterized by vasoconstriction or neuropathic pain in a subject in need thereof, the method comprising (a) determining that the disease or disorder is associated with vasoconstriction or neuropathic pain; and (b) administering to the subject a therapeutically effective amount of a dual N-type and L-type calcium channel blocker selective for the N-type calcium channel.

In some embodiments, the disease or disorder characterized by vasoconstriction or neuropathic pain is characterized by neuropathic pain (e.g., chronic neuropathic pain), vasoconstriction, dysesthetic pain, hyperesthetic pain, allodynia, lancinating pain, crampy pain, dull pain, burning pain, body temperature changes of the subject, hyperesthesia, changes in skin or tissue color, edema, changes in skin turgor, rubor, pallor, cyanosis, vasospasm, or any combination thereof. In certain embodiments, the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence. In certain of these embodiments, the disease or disorder is Raynaud's syndrome. For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In some embodiments, the subject is also diagnosed with hypertension; and wherein after administration of the dual N-type and L-type calcium channel blocker selective for the N-type calcium channel to the subject, the blood pressure (e.g., the systolic blood pressure) of the subject is reduced.

In some embodiments, the subject also has hypertension; and wherein after administration of the dual N-type and L-type calcium channel blocker selective for the N-type calcium channel to the subject, the blood pressure (e.g., the systolic blood pressure) of the subject is reduced. In some embodiments, the subject does not have hypertension; and wherein after administration of the dual N-type and L-type calcium channel blocker selective for the N-type calcium channel to the subject, the blood pressure (e.g., the systolic blood pressure) of the subject is not reduced. Without wishing to be bound by theory, it is believed that when the subject has hypertension, the dual N-type and L-type calcium channel blocker selective for the N-type calcium channel reduces the blood pressure of the subject; however, when the subject does not have hypertension (i.e., the subject is normotensive), the dual N-type and L-type calcium channel blocker selective for the N-type calcium channel does not reduce the blood pressure of the subject. In some embodiments, after administration of the dual N-type and L-type calcium channel blocker selective for the N-type calcium channel to the subject, the cardiac function of the subject is improved (e.g., left ventricular function of the subject is improved).

In some embodiments, after administration of the dual N-type and L-type calcium channel blocker selective for the N-type calcium channel to the subject, the cardiac function of the subject is improved. In some embodiments, improving the cardiac function in the subject comprises improving the left ventricular function of the subject. In some embodiments, the subject has hypertension. In some embodiments, the subject does not have hypertension.

In some embodiments, the subject has hypertension; and after administration of the dual N-type and L-type calcium channel blocker selective for the N-type calcium channel to the subject, the blood pressure of the subject is decreased and the cardiac function (e.g., left ventricular function) of the subject is improved.

In some embodiments, the subject does not have hypertension; and after administration of the dual N-type and L-type calcium channel blocker selective for the N-type calcium channel to the subject, the blood pressure of the subject is not decreased and the cardiac function (e.g., left ventricular function) of the subject is improved.

In some embodiments, the subject has hypertension and osteoporosis; and after administration of the dual N-type and L-type calcium channel blocker selective for the N-type calcium channel to the subject, the blood pressure of the subject is decreased and the bone density in the subject is increased.

In some embodiments, the subject does not have hypertension; the subject has osteoporosis; and after administration of the dual N-type and L-type calcium channel blocker selective for the N-type calcium channel to the subject, the blood pressure of the subject is not decreased and the bone density in the subject is increased.

In some embodiments, the subject has hypertension; and after administration of the dual N-type and L-type calcium channel blocker selective for the N-type calcium channel to the subject, the blood pressure of the subject is decreased and the bone density in the subject is increased.

In some embodiments, the subject does not have hypertension; and after administration of the dual N-type and L-type calcium channel blocker selective for the N-type calcium channel to the subject, the blood pressure of the subject is not decreased and the bone density in the subject is increased.

In some embodiments, the subject has hypertension and atherosclerosis; and after administration of the dual N-type and L-type calcium channel blocker selective for the N-type calcium channel to the subject, the blood pressure of the subject is decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject does not have hypertension; the subject has atherosclerosis; and after administration of the dual N-type and L-type calcium channel blocker selective for the N-type calcium channel to the subject, the blood pressure of the subject is not decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has hypertension; and after administration of the dual N-type and L-type calcium channel blocker selective for the N-type calcium channel to the subject, the blood pressure of the subject is decreased and renal function in the subject is improved.

In some embodiments, the subject does not have hypertension; and after administration of the dual N-type and L-type calcium channel blocker selective for the N-type calcium channel to the subject, the blood pressure of the subject is not decreased and renal function in the subject is improved.

In some embodiments, the subject has hypertension; and after administration of the dual N-type and L-type calcium channel blocker selective for the N-type calcium channel to the subject, the blood pressure of the subject is decreased and renal function in the subject is improved. In some embodiments, the subject does not have hypertension; and after administration of the dual N-type and L-type calcium channel blocker selective for the N-type calcium channel to the subject, the blood pressure of the subject is not decreased and renal function in the subject is improved.

In some embodiments, the subject has hypertension; the subject was previously treated with antihypertensive agents before administration of the dual N-type and L-type calcium channel blocker selective for the N-type calcium channel; and after administration of the dual N-type and L-type calcium channel blocker selective for the N-type calcium channel to the subject, the blood pressure of the subject is decreased.

In some embodiments, the subject does not have hypertension; the subject was previously treated with antihypertensive agents before administration of the dual N-type and L-type calcium channel blocker selective for the N-type calcium channel; and after administration of the dual N-type and L-type calcium channel blocker selective for the N-type calcium channel to the subject, the blood pressure of the subject is is not decreased.

In some embodiments, the subject has hypertension; the subject has scleroderma; the subject has a digital ulcer; and after administration of the dual N-type and L-type calcium channel blocker selective for the N-type calcium channel to the subject, the blood pressure of the subject is decreased, and the digital ulcer is treated. In some embodiments, treating the digital ulcer comprises healing or improving the condition of the digital ulcer.

In some embodiments, the subject does not have hypertension; the subject has scleroderma; the subject has a digital ulcer; and after administration of the dual N-type and L-type calcium channel blocker selective for the N-type calcium channel to the subject, the blood pressure of the subject is not reduced, and the digital ulcer is treated. In some embodiments, treating the digital ulcer comprises healing or improving the condition of the digital ulcer.

In some embodiments, the subject has hypertension; the subject has scleroderma; the disease or disorder associated with dysregulation of blood flow and sympathetic nervous system overactivity is Raynaud's syndrome; and after administration of the dual N-type and L-type calcium channel blocker selective for the N-type calcium channel to the subject, the blood pressure of the subject is decreased.

In some embodiments, the subject does not have hypertension; the subject has scleroderma; the disease or disorder associated with dysregulation of blood flow and sympathetic nervous system overactivity is Raynaud's syndrome; and after administration of the dual N-type and L-type calcium channel blocker selective for the N-type calcium channel to the subject, the blood pressure of the subject is not reduced.

In some embodiments, the blood pressure (e.g., systolic blood pressure) of the subject is reduced by greater than 1 mm Hg. For example, the systolic blood pressure of the subject is reduced by greater than 2, 5, 8, 10, 12, 15, 20, 25, or 30 mm Hg. For example, the systolic blood pressure of the subject is reduced by about 1-5 mm Hg, about 5-10 mm Hg, about 10-15 mm Hg, about 15-20 mm Hg, or about 20-30 mm Hg.

In some embodiments, the subject is also being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject has lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the treatment for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereofcomprises administering a therapeutic agent. Therapeutic agents known in the art for treating lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereofcan be found in, e.g., the Physicians' desk reference. (71st ed.). (2017). Montvale, NJ: PDR Network, which is incorporated by reference herein in its entirety.

In certain embodiments, the subject is being treated for scleroderma. For example, the subject is being treated for scleroderma with interstitial lung disease. In some embodiments, the subject has lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the method further comprises administering an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the method further comprises administering nintedanib. In some embodiments, the method further comprises administering a calcineurin inhibitor, a non-steroidal anti-inflammatory drug, or both. In some embodiments, the dual N-type and L-type calcium channel blocker selective for the N-type calcium channel, the calcineurin inhibitor, and the non-steroidal anti-inflammatory drug are administered separately, sequentially, or simultaneously. In some embodiments, the dual N-type and L-type calcium channel blocker selective for the N-type calcium channel, the calcineurin inhibitor, and the non-steroidal anti-inflammatory drug are administered separately. In some embodiments, the dual N-type and L-type calcium channel blocker selective for the N-type calcium channel, the calcineurin inhibitor, and the non-steroidal anti-inflammatory drug are administered sequentially. In some embodiments, the dual N-type and L-type calcium channel blocker selective for the N-type calcium channel, the calcineurin inhibitor, and the non-steroidal anti-inflammatory drug are administered simultaneously. In some embodiments, the dual N-type and L-type calcium channel blocker selective for the N-type calcium channel, the calcineurin inhibitor, and the non-steroidal anti-inflammatory drug are administered simultaneously as a fixed dosage form. In some embodiments, the calcineurin inhibitor is a cyclosporine. In some embodiments, the non steroidal anti-inflammatory drug is aspirin.

In certain embodiments, the subject is being treated for lupus. In certain embodiments, the subject is diagnosed with lupus. In certain embodiments, the subject has lupus. In some embodiments, the method further comprises administering an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the subject is being treated for rheumatoid arthritis. In certain embodiments, the subject has rheumatoid arthritis. In certain embodiments, the subject is diagnosed with rheumatoid arthritis. In some embodiments, the method further comprises administering an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the subject is being treated for Sjögren's syndrome. In certain embodiments, the subject has Sjögren's syndrome. In certain embodiments, the subject is diagnosed with Sjögren's syndrome. In some embodiments, the method further comprises administering an agent selected from the group consisting of: plaquenil, an antimalarial drug (e.g., hydroxychloroquine), evoxac, cevimeline, infliximab, or any combination thereof.

In certain embodiments, the subject is being treated for idiopathic pulmonary fibrosis. In certain embodiments, the subject has idiopathic pulmonary fibrosis. In certain embodiments, the subject is diagnosed with idiopathic pulmonary fibrosis. In some embodiments, the method further comprises administering an agent selected from the group consisting of: nintedanib, pirfenidone, or any combination thereof.

In some embodiments, the disease or disorder characterized by vasoconstriction or neuropathic pain in a subject is secondary Raynaud's disease; the subject is also being treated for scleroderma; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder characterized by vasoconstriction or neuropathic pain in a subject is secondary Raynaud's disease; the subject is also being treated for scleroderma with interstitial lung disease; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder characterized by vasoconstriction or neuropathic pain in a subject is secondary Raynaud's disease; the subject is also being treated for lupus; the dual N-type and L-type calcium channel blocker selective for the N-type calcium channel is cilnidipine; and the subject is further administered an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In some embodiments, the disease or disorder characterized by vasoconstriction or neuropathic pain in a subject is secondary Raynaud's disease; the subject is also being treated for rheumatoid arthritis; the dual N-type and L-type calcium channel blocker selective for the N-type calcium channel is cilnidipine; and the subject is further administered an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In some embodiments, the disease or disorder characterized by vasoconstriction or neuropathic pain in a subject is secondary Raynaud's disease; the subject is also being treated for Sjögren's syndrome; the dual N-type and L-type calcium channel blocker selective for the N-type calcium channel is cilnidipine; and the subject is further administered an agent selected from the group consisting of: plaquenil, an antimalarial drug (e.g., hydroxychloroquine), evoxac, cevimeline, infliximab, or any combination thereof.

In some embodiments, the disease or disorder characterized by vasoconstriction or neuropathic pain in a subject is secondary Raynaud's disease; the subject is also being treated for idiopathic pulmonary fibrosis; the dual N-type and L-type calcium channel blocker selective for the N-type calcium channel is cilnidipine; and the subject is further administered an agent selected from the group consisting of: nintedanib, pirfenidone, or any combination thereof.

In some embodiments, the method comprises administering at least one additional therapeutic agent to the subject. The at least one additional therapeutic agent can be administered simultaneously, separately, sequentially, or in combination (e.g., for more than two agents) with the dual N-type and L-type calcium channel blocker selective for the N-type calcium channel and/or Nav 1.7 sodium channel blocker. Non-limiting examples of additional therapeutic agents include calcium channel blockers, sodium channel blockers (e.g., Nav 1.7 sodium channel blocker), and therapeutic agents that relieve pain.

In certain embodiments, the at least one additional therapeutic agent is selected from the group consisting of: riociguat, amlodipine, nifedipine, nicardipine, conotoxins, cadmium, caroverine, gabapentin, levetiracetam, lamotrigine, NP078585, pregabalin, TROX-1, acetaminophen, non-steroidal anti-inflammatory agents (e.g., ibuprofen), and ziconotide.

In certain embodiments, the at least one additional therapeutic agent is selected from the group consisting of: oxycodone, tramadol, Dilaudid, OxyContin, Cymbalta, a statin, gabapentin, pregabalin, an angiotensin-converting-enzyme (ACE) inhibitor, an angiotensin receptor blocker (ARB), niacin, a proton pump inhibitor, aspirin, Fentanyl Transdermal System, acetaminophen/oxycodone, Roxicodone, Ultram, hydromorphone, Percocet, MS Contin, Butrans, morphine, hydromorphone, methadone, buprenorphine, duloxetine, fentanyl, Duragesic, Endocet, Roxanol, Kadian, Roxicet, ConZip, Methadose, Oxyfast, Dazidox, Fentora, Irenka, Methadone Diskets, Oramorph SR, Roxicodone Intensol, Xtampza ER, Actiq, Belbuca, ETH-Oxydose, Infumorph, naloxone / pentazocine, Oxaydo, Oxydose, OxyIR, ziconotide, Abstral, Astramorph PF, Buprenex, Dolophine, Duramorph, Duramorph PF, Embeda, Lazanda, MorphaBond ER, morphine/naltrexone, Prialt, RMS, Roxanol-T, Sublimaze, Subsys, Talwin Nx, Magnacet, Nalocet, Narvox, Perloxx, Primlev, Xolox, and Prolate.

In another aspect, disclosed herein is a method of treating secondary Raynaud's syndrome in a subject in need thereof, the method comprising administering to the subject (a) a therapeutically effective amount of a dual N-type and L-type calcium channel blocker selective for the N-type calcium channel, and (b) at least one additional therapeutic agent.

Without wishing to be bound by theory, it is believed that the secondary Raynaud's syndrome is a consequence of another disease or disorder (also referred to herein as a "primary" disease or disorder). In some embodiments, the primary disease or disorder is lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the at least one additional therapeutic agent is an accepted treatment for the primary disease or disorder.

In certain embodiments, the primary disorder is scleroderma. For example, the primary disorder is scleroderma with interstitial lung disease. In some of embodiments, the method further comprises administering an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the method further comprises administering nintedanib. In some embodiments, the method further comprises administering a calcineurin inhibitor, a non-steroidal anti-inflammatory drug, or both. In some embodiments, the dual N-type and L-type calcium channel blocker selective for the N-type calcium channel, the calcineurin inhibitor, and the non-steroidal anti-inflammatory drug are administered separately, sequentially, or simultaneously. In some embodiments, the dual N-type and L-type calcium channel blocker selective for the N-type calcium channel, the calcineurin inhibitor, and the non-steroidal anti-inflammatory drug are administered separately. In some embodiments, the dual N-type and L-type calcium channel blocker selective for the N-type calcium channel, the calcineurin inhibitor, and the non-steroidal anti-inflammatory drug are administered sequentially. In some embodiments, the dual N-type and L-type calcium channel blocker selective for the N-type calcium channel, the calcineurin inhibitor, and the non-steroidal anti-inflammatory drug are administered simultaneously. In some embodiments, the dual N-type and L-type calcium channel blocker selective for the N-type calcium channel, the calcineurin inhibitor, and the non-steroidal anti-inflammatory drug are administered simultaneously as a fixed dosage form. In some embodiments, the calcineurin inhibitor is a cyclosporine. In some embodiments, the non steroidal anti-inflammatory drug is aspirin.

In certain embodiments, the primary disorder is lupus. In some embodiments, the method further comprises administering an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the primary disorder is rheumatoid arthritis. In some embodiments, the method further comprises administering an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the primary disorder is Sjögren's syndrome. In some embodiments, the method further comprises administering an agent selected from the group consisting of: plaquenil, an antimalarial drug (e.g., hydroxychloroquine), evoxac, cevimeline, infliximab, or any combination thereof.

In another aspect, disclosed herein is a method of reducing a sensation of burning pain, paresthesia, dysesthesia, hypoesthesia, allodynia, or hyperesthesia (e.g., burning pain) in a subject, comprising administering a therapeutically effective amount of a dual N-type and L-type calcium channel blocker selective for the N-type calcium channel to a subject. In some embodiments, the sensation of burning pain, paresthesia, dysesthesia, hypoesthesia, allodynia, or hyperesthesia is a symptom of a disease or disorder associated with dysregulation of blood flow and sympathetic nervous system overactivity. In some embodiments, the sensation of burning pain, paresthesia, dysesthesia, hypoesthesia, allodynia, or hyperesthesia in a subject is a symptom of a disease or disorder characterized by neuropathic pain and/or vasoconstriction. A sensation of burning pain can be measured using one or more of the following: the Galer neuropathic pain scale, the ID pain Scale, NPQ, PainDETECT, LANS S, DN4 scales, and/or the Standardized Evaluation of Pain (StEP) tool. See, for example, Cruccu G, Truini A. Tools for assessing neuropathic pain. PLoS Med. 2009;6(4):e1000045. doi: 10.1371/journal.pmed. 1000045.

In some embodiments, the dual N-type and L-type calcium channel blocker selective for the N-type calcium channel is selected from the group consisting of: cilnidipine, Z-160, zicinotide, ralfinamide, CNV2197944, or pharmaceutically acceptable salts thereof. In some embodiments, the dual N-type and L-type calcium channel blocker selective for the N-type calcium channel is selected from the group consisting of: cilnidipine, Z-160, ralfinamide, CNV2197944, or pharmaceutically acceptable salts thereof. In some embodiments of the methods disclosed herein, the dual N-type and L-type calcium channel blocker selective for the N-type calcium channel is selected from the group consisting of: cilnidipine, Z-160, CNV2197944, or pharmaceutically acceptable salts thereof. In some embodiments of the methods disclosed herein, the dual N-type and L-type calcium channel blocker selective for the N-type calcium channel is selected from the group consisting of: cilnidipine, Z-160, zicinotide, or pharmaceutically acceptable salts thereof. In certain embodiments, the dual N-type and L-type calcium channel blocker selective for the N-type calcium channel is a dihydropyridine N-type calcium channel blocker. In certain of these embodiments, the dual N-type and L-type calcium channel blocker selective for the N-type calcium channel is cilnidipine or a pharmaceutically acceptable salt thereof. In some embodiments, the dual N-type and L-type calcium channel blocker selective for the N-type calcium channel is CNV2197944

In some embodiments, the dosage of the dual N-type and L-type calcium channel blocker selective for the N-type calcium channel is about 0.003 mg/kg to about 5 mg/kg. For example, the dosage of the dual N-type and L-type calcium channel blocker selective for the N-type calcium channel is about 0.005 mg/kg to about 2 mg/kg, about 0.01 mg/kg to about 1 mg/kg, 0.03 mg/kg to about 0.9 mg/kg, 0.06 mg/kg to about 0.3 mg/kg, about 0.1 mg/kg to about 0.18 mg/kg, or about 1 mg/kg to about 2 mg/kg. In some embodiments, the dosage of the dual N-type and L-type calcium channel blocker selective for the N-type calcium channel is from about 5 mg to about 50 mg, for example, from about 5 mg to about 15 mg, from about 15 mg to about 25 mg, from about 5 mg to about 25 mg, about 10 mg, or about 20 mg.

Without wishing to be bound by theory, it is believed that dual N-type and L-type calcium channel blocker selective for the N-type calcium channels may decrease the blood pressure (e.g., the systolic blood pressure) of subjects that are hypertensive. As such, it may be beneficial to administer an agent that increases blood pressure (e.g., the systolic blood pressure) in combination with the dual N-type and L-type calcium channel blocker selective for the N-type calcium channel. In some embodiments of the methods disclosed herein, the method further comprises administering to the subject a therapeutically effective amount of an agent that increases blood pressure (e.g., the systolic blood pressure). In certain embodiments, the agent that increases blood pressure (e.g., the systolic blood pressure) is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines. In certain embodiments, the blood pressure (e.g., the systolic blood pressure) of the subject before and after administration of the dual N-type and L-type calcium channel blocker selective for the N-type calcium channel and the agent that increases blood pressure (e.g., the systolic blood pressure) is substantially the same.

In some embodiments of the methods disclosed herein, the dual N-type and L-type calcium channel blocker selective for the N-type calcium channel is administered orally, parenterally, transdermally, by inhalation, intranasally, sublingually, neuraxially, or ocularly

In some embodiments of the methods disclosed herein, the bone density of the subject does not decrease. In some of these embodiments, the bone density of the subject increases. This may occur through a reduction in the number of osteoclasts in the subject and/or an increase in the ratio of alkaline phosphate to tartrate resistant acid phosphatase (TRAP).

In some embodiments of the methods disclosed herein, the method further comprises selecting a subject identified or diagnosed as having reduced bone density for the treatment. In certain embodiments, the subject identified or diagnosed as having reduced bone density is afflicted with osteoporosis. In certain of these embodiments, the subject is female. In some embodiments, after administration of the dual N-type and L-type calcium channel blocker selective for the N-type calcium channel to the subject, bone density in the subject is increased. Without wishing to be bound by theory, the increase in bone density occurs by means of bone resorption. In some embodiments, after administration of the dual N-type and L-type calcium channel blocker selective for the N-type calcium channel to the subject, osteoporosis in the subject is improved.

In some embodiments of the methods disclosed herein, the method further comprises selecting a subject identified or diagnosed as having reduced renal function for the treatment. In certain embodiments, the renal function of the subject is not reduced after treatment. In certain embodiments, the renal function of the subject is improved after treatment.

In some embodiments, each administration of the dual N-type and L-type calcium channel blocker selective for the N-type calcium channel is separated by at least about 12 hours. For example, each administration of the dual N-type and L-type calcium channel blocker selective for the N-type calcium channel is separated by at least about 24 hours, at least about 30 hours, at least about 48 hours, at least about 60 hours, at least about 72 hours, at least about 4 days, at least about 5 days, at least about 6 days, at least about 1 week, at least about 9 days, at least about 12 days, or at least about 2 weeks.

In some embodiments, the dual N-type and L-type calcium channel blocker selective for the N-type calcium channel causes sympathetic tone diminution, direct smooth muscle relaxation, dysesthetic pain, burning pain, body temperature changes of the subject, hyperesthesia, changes in skin or tissue color, edema, changes in skin turgor, ruble, pallor, cyanosis, vasospasm, or any combination thereofin the subject.

In some embodiments, the dual N-type and L-type calcium channel blocker selective for the N-type calcium channel exhibits at least a 5-fold selectivity for the N-type calcium channel over an L-type calcium channel. For example, the dual N-type and L-type calcium channel blocker selective for the N-type calcium channel exhibits at least a 10-fold, at least a 30-fold, at least a 50-fold, at least a 80-fold, at least a 100-fold, at least a 300-fold, at least a 500-fold, at least a 800-fold, at least a 900-fold, or at least a 1000-fold selectivity for the N-type calcium channel over an L-type calcium channel. For example, the dual N-type and L-type calcium channel blocker selective for the N-type calcium channel exhibits at least a 50-fold to 100-fold selectivity for the N-type calcium channel over an L-type calcium channel.

In another aspect, disclosed herein is a method of reducing pain or discomfort in a subject caused by a reduction of body temperature in the subject, comprising administering an effective amount of a dual N-type and L-type calcium channel blocker selective for the N-type calcium channel to the subject, wherein the reduction of body temperature in the subject is caused by an exposure of the subject to air having a temperature of less than 25°C.

In some embodiments, vasoconstriction in the subject is reduced after administering the dual N-type and L-type calcium channel blocker selective for the N-type calcium channel.

In some embodiments, the reduction of body temperature in the subject is caused by an exposure of the subject to air having a temperature of less than 20°C, for example, less than 20 °C, less than 15 °C, less than 10 °C, less than 5 °C, less than 0 °C, less than -5 °C, less than -10 °C, or less than -5 °C.

In some embodiments, the pain or discomfort that is reduced occurs during the exposure of the subject to air having a temperature of less than 25°C.

In some embodiments, the reduction of body temperature in the subject is followed by a restoration to the normal body temperature in the subject, and the pain or discomfort that is reduced occurs after restoration to the normal body temperature in the subject.

In some embodiments, the reduction of body temperature in the subject comprises reduction in the temperature of a region of the body of the subject. In some embodiments, the reduction of body temperature in the subject comprises a reduction in the temperature of a digit, an extremity, or alar circulatory region of the subject. In some embodiments, the reduction of body temperature in the subject comprises reduction in the temperature of a hand or foot of the subject. For example, in some embodiments, the reduction of body temperature in the subject comprises reduction in the temperature of a hand of the subject. For example, the reduction of body temperature in the subject comprises reduction in the temperature of a finger of the subject. In some embodiments, the reduction of body temperature in the subject comprises reduction in the temperature of a foot of the subject.

In another aspect, disclosed herein is a method of reducing susceptibility of a subject to cold-induced pain or discomfort, comprising: administering an effective amount of a dual N-type and L-type calcium channel blocker selective for the N-type calcium channel to the subject.

In another aspect, disclosed herein is a method of treating cold-induced pain or discomfort, comprising: administering an effective amount of a dual N-type and L-type calcium channel blocker selective for the N-type calcium channel to the subject.

In some embodiments, the subject experiences a lesser degree of the pain or discomfort upon exposure to air having a temperature of less than 25 °C than as compared to a subject that is not administered a dual N-type and L-type calcium channel blocker selective for the N-type calcium channel and is exposed to air having a temperature of less than 25°C. For example, the subject experiences a lesser degree of the pain or discomfort upon exposure to air having a temperature of less than 20 °C than as compared to a subject that is not administered a dual N-type and L-type calcium channel blocker selective for the N-type calcium channel and is exposed to air having a temperature of less than 20 °C. For example, the subject experiences a lesser degree of the pain or discomfort upon exposure to air having a temperature of less than 15 °C than as compared to a subject that is not administered a dual N-type and L-type calcium channel blocker selective for the N-type calcium channel and is exposed to air having a temperature of less than 15 °C. For example, the subject experiences a lesser degree of the pain or discomfort upon exposure to air having a temperature of less than 10 °C than as compared to a subject that is not administered a dual N-type and L-type calcium channel blocker selective for the N-type calcium channel and is exposed to air having a temperature of less than 10 °C. For example, the subject experiences a lesser degree of the pain or discomfort upon exposure to air having a temperature of less than 5 °C than as compared to a subject that is not administered a dual N-type and L-type calcium channel blocker selective for the N-type calcium channel and is exposed to air having a temperature of less than 5 °C. For example, the subject experiences a lesser degree of the pain or discomfort upon exposure to air having a temperature of less than 0 °C than as compared to a subject that is not administered a dual N-type and L-type calcium channel blocker selective for the N-type calcium channel and is exposed to air having a temperature of less than 0 °C. For example, the subject experiences a lesser degree of the pain or discomfort upon exposure to air having a temperature of less than -10 °C than as compared to a subject that is not administered a dual N-type and L-type calcium channel blocker selective for the N-type calcium channel and is exposed to air having a temperature of less than -10 °C.

In some embodiments, the dual N-type and L-type calcium channel blocker selective for the N-type calcium channel is selected from the group consisting of: cilnidipine, Z-160, zicinotide, ralfinamide, CNV2197944, or pharmaceutically acceptable salts thereof. In some embodiments, the dual N-type and L-type calcium channel blocker selective for the N-type calcium channel is selected from the group consisting of: cilnidipine, Z-160, ralfinamide, CNV2197944, or pharmaceutically acceptable salts thereof. In some embodiments, the dual N-type and L-type calcium channel blocker selective for the N-type calcium channel is selected from the group consisting of: cilnidipine, Z-160, CNV2197944, or pharmaceutically acceptable salts thereof. In some embodiments, the dual N-type and L-type calcium channel blocker selective for the N-type calcium channel is CNV2197944.

For example, the dual N-type and L-type calcium channel blocker selective for the N-type calcium channel is cilnidipine.

In another aspect, disclosed herein is a method of treating sickle cell disease in a subject in need thereof, comprising administering an effective amount of a dual N-type and L-type calcium channel blocker selective for the N-type calcium channel to the subj ect.

In another aspect, disclosed herein is a method of selecting a patient for treatment including administration of a therapeutically effective amount of a dual N-type and L-type calcium channel blocker selective for the N-type calcium channel, the method comprising:
identifying a patient having sickle cell disease; and
selecting the patient for treatment including administration of a therapeutically effective amount of a compound of a dual N-type and L-type calcium channel blocker selective for the N-type calcium channel.

In some embodiments, the dual N-type and L-type calcium channel blocker selective for the N-type calcium channel is selected from the group consisting of: cilnidipine, Z-160, CNV2197944, or pharmaceutically acceptable salts thereof. In some embodiments, the dual N-type and L-type calcium channel blocker selective for the N-type calcium channel is cilnidipine.

In some embodiments, the sickle cell disease is associated with an SS genotype. In some embodiments, the sickle cell disease is associated with an SC genotype.

In some embodiments, the treating comprises decreasing the severity of one or more symptoms. In some embodiments, the treating comprises decreasing the frequency of one or more symptoms. In some embodiments, the treating comprises decreasing the duration of one or more symptoms.

In some embodiments, the one or more symptoms is selected from the group consisting of: anemia, fatigue, pain, swelling (e.g., swelling of hands and/or feet), infection, delayed growth, impaired vision, or any combination thereof. In some embodiments, the one or more symptoms includes pain.

In some embodiments, the method further comprises preventing or reducing the severity of one or more diseases or disorders associated with sickle cell disease selected from the group consisting of: bacterial infections, ischemic stroke, hemorrhagic stroke, silent stroke, cholelithiasis, cholecystitis, deterioration of the bones, hyposplenism, priapism, osteomyelitis, acute papillary necrosis, leg ulcers, an eye disorder, pulmonary hypertension, and a kidney disorder. In some embodiments, the deterioration of the bones comprises avascular necrosis. In some embodiments, the eye disorder is selected from the group consisting of: background retinopathy, proliferative retinopathy, vitreous haemorrhages, and retinal detachments. In some embodiments, the kidney disorder is chronic kidney failure.

In some embodiments, the method further comprises administering a second therapeutic agent selected from the group consisting of: hydroxyurea, L-glutamine, crizanlizumab, voxelotor, and senicapoc.

In another aspect, disclosed herein is a method of treating vasculitis in a subject in need thereof, comprising administering an effective amount of a dual N-type and L-type calcium channel blocker selective for the N-type calcium channel to the subject.

In another aspect, disclosed herein is a method of selecting a patient for treatment including administration of a therapeutically effective amount of a dual N-type and L-type calcium channel blocker selective for the N-type calcium channel, the method comprising:
identifying a patient having vasculitis; and
selecting the patient for treatment including administration of a therapeutically effective amount of a compound of a dual N-type and L-type calcium channel blocker selective for the N-type calcium channel.

In some embodiments, the vasculitis is selected from large vessel vasculitis, medium vessel vasculitis, and small vessel vasculitis. In some embodiments, the vasculitis is large vessel vasculitis. In some embodiments, the large vessel vasculitis is Takayasu arteritis. In some embodiments, the vasculitis is medium vessel vasculitis. In some embodiments, the medium vessel vasculitis is Buerger's disease or polyarteritis nodosa. In some embodiments, the vasculitis is small vessel vasculitis. In some embodiments, the small vessel vasculitis is selected from granulomatosis with polyangiitis, Churg-Strauss syndrome, and microscopic polyangiitis.

In some embodiments, the method further comprises reducing inflammation in a blood vessel in the subject.

In some embodiments, the method further comprises fully or partially restoring normal elasticity of a blood vessel in the subject. In some embodiments, the method further comprises fully restoring normal elasticity of a blood vessel in the subject. In some embodiments, the method further comprises partially restoring normal elasticity of a blood vessel in the subject.

In some embodiments, the method further comprises changing the diameter of a blood vessel in the subject. In some embodiments, the method further comprises increasing the diameter of a blood vessel in the subject. In some embodiments, the method further comprises decreasing the diameter of a blood vessel in the subject. In some embodiments, the blood vessel is an artery. In some embodiments, the blood vessel is a vein. In some embodiments, the blood vessel is a capillary.

In some embodiments, the method further comprises alleviating one or more symptoms of vasculitis and/or disorders associated with vasculitis in the subject selected from the group consisting of: fever, weight loss, purpura, livedo reticularis, muscle pain, muscle inflammation, joint pain, joint swelling, mononeuritis multiplex, headache, stroke, tinnitus, reduced visual acuity, acute visual loss, high blood pressure, gangrene, nose bleeds, bloody cough, lung infiltrates, abdominal pain, bloody stool, a perforation in the gastrointestinal tract, inflammation of a kidney (e.g., inflammation of glomeruli).

In some embodiments, the method further comprises decreasing the number or size of petechia and/or purpura in the subject.

In some embodiments, the underlying cause of the vasculitis is infection (e.g., viral infection). In some embodiments, the underlying cause of the vasculitis is leukocyte migration. In some embodiments, the underlying cause of the vasculitis is adenosine deaminase 2 deficiency. In some embodiments, the underlying cause of the vasculitis is haploinsufficiency of A20.

In another aspect, disclosed herein is a method of treating thrombosis in a subject in need thereof, comprising administering an effective amount of a dual N-type and L-type calcium channel blocker selective for the N-type calcium channel to the subject.

In another aspect, disclosed herein is a method of selecting a patient for treatment including administration of a therapeutically effective amount of a dual N-type and L-type calcium channel blocker selective for the N-type calcium channel, the method comprising:
identifying a patient having thrombosis; and
selecting the patient for treatment including administration of a therapeutically effective amount of a compound of a dual N-type and L-type calcium channel blocker selective for the N-type calcium channel.

In some embodiments, the thrombosis is venous thrombosis or arterial thrombosis. In some embodiments, the thrombosis is selected from deep vein thrombosis, portal vein thrombosis, renal vein thrombosis, jugular vein thrombosis, Budd-Chiari syndrome, Paget-Schroetter disease, cerebral venous sinus thrombosis, thrombotic stroke, and myocardial infarction.

In some embodiments, an underlying cause of the thrombosis is thrombophilia. In some embodiments, the subject has a reduced amount of procoagulant and/or anticoagulant. In some embodiments, the treating comprises increasing the amount of procoagulant in the subject. In some embodiments, the treating comprises increasing the amount of procoagulant in the subject by about 1% to about 50% (e.g., by about 1% to about 5%, by about 5% to about 10%, by about 10% to about 15%, by about 15% to about 20%, by about 20% to about 25%, by about 25% to about 30%, by about 30% to about 40%, or by about 40% to about 50%).In some embodiments, the treating comprises increasing the amount of anticoagulant in the subject. In some embodiments, the treating comprises increasing the amount of anticoagulant in the subject by about 1% to about 50% (e.g., by about 1% to about 5%, by about 5% to about 10%, by about 10% to about 15%, by about 15% to about 20%, by about 20% to about 25%, by about 25% to about 30%, by about 30% to about 40%, or by about 40% to about 50%).

In some embodiments, an underlying cause of the thrombosis is damage to the endothelium of a blood vessel. In some embodiments, the method further comprises repairing the damage to the endothelium. In some embodiments, the method further comprises preventing damage to the endothelium.

In some embodiments, an underlying cause of the thrombosis is reduced blood flow. In some embodiments, the reduced blood flow occurs in an extremity of the subject (e.g., an arm or a leg). In some embodiments, the method further comprises increasing blood flow in the subject.

In some embodiments, one or more additional therapeutic agents are administered to the subject. In some embodiments, the one or more additional therapeutic agents are selected from the group consisting of warfarin, rivaroxaban, apixaban, heparin, enoxaparin, apixaban, dabigatran, fondaparinux, urokinase, and edoxaban.

In another aspect, disclosed herein is a method of treating a kidney disorder in a subject in need thereof, comprising administering an effective amount of a dual N-type and L-type calcium channel blocker selective for the N-type calcium channel to the subject, wherein the kidney disorder is a complication of a disease or disorder.

In another aspect, disclosed herein is a method of selecting a patient for treatment including administration of a therapeutically effective amount of a dual N-type and L-type calcium channel blocker selective for the N-type calcium channel, the method comprising:
identifying a patient having a kidney disorder; and
selecting the patient for treatment including administration of a therapeutically effective amount of a compound of a dual N-type and L-type calcium channel blocker selective for the N-type calcium channel.

In some embodiments, the kidney disorder is selected from chronic kidney disease, kidney stones, glomerulonephritis, polycystic kidney disease, and urinary tract infection.

In some embodiments, an underlying cause of the kidney disorder is reduced blood flow to the kidneys. In some embodiments, the treating further comprises increasing blood flow to the kidneys.

In another aspect, disclosed herein is a method of treating hypertension and chronic kidney disease in a subject in need thereof, comprising administering an effective amount of a dual N-type and L-type calcium channel blocker selective for the N-type calcium channel to the subject.

In another aspect, disclosed herein is a method of selecting a patient for treatment including administration of a therapeutically effective amount of a dual N-type and L-type calcium channel blocker selective for the N-type calcium channel, the method comprising:
identifying a patient having hypertension and chronic kidney disease; and
selecting the patient for treatment including administration of a therapeutically effective amount of a compound of a dual N-type and L-type calcium channel blocker selective for the N-type calcium channel.

In some embodiments, the treating further comprises improving blood flow to the kidneys of the subject.

In some embodiments, the subject is undergoing dialysis support, and wherein the subject requires less dialysis support than if the subject were not administered an effective amount of a dual N-type and L-type calcium channel blocker selective for the N-type calcium channel.

In some embodiments, the method further comprises administering a second therapeutic agent selected from the group consisting of: azilsartan, candesartan, eprosartan, atorvastatin, fluvastatin, lovastatin, pravastatin, simvastatin, rosuvastatin, erythropoietin chlorothiazide, chlorthalidone, bumetanide, vitamin D, or calcium supplements.

In some embodiments, the dual N-type and L-type calcium channel blocker selective for the N-type calcium channel is selected from the group consisting of: cilnidipine, Z-160, CNV2197944, or pharmaceutically acceptable salts thereof.

In some embodiments, the dual N-type and L-type calcium channel blocker selective for the N-type calcium channel is cilnidipine.

In some embodiments (e.g., any embodiment disclosed herein), the dual N-type and L-type calcium channel blocker selective for the N-type calcium channel further inhibits a sodium channel. In certain embodiments, the sodium channel is a Nav 1.7 sodium channel. In some embodiments, the dual N-type and L-type calcium channel blocker selective for the N-type calcium channel that further inhibits a sodium channel is cilnidipine. In some embodiments, the dual N-type and L-type calcium channel blocker selective for the N-type calcium channel that further inhibits a Nav 1.7 sodium channel is more effective at treating the disease or disorder associated with dysregulation of blood flow and sympathetic nervous system overactivity than an equivalent dose of a non-N-selective calcium channel blocker useful to treat the disease or disorder. In some embodiments, the dual N-type and L-type calcium channel blocker selective for the N-type calcium channel that further inhibits a Nav 1.7 sodium channel is more effective at treating the disease or disorder characterized by neuropathic pain or vasoconstriction than an equivalent dose of a non-N-selective calcium channel blocker useful to treat the disease or disorder.

In some embodiments, after administration of the dual N-type and L-type calcium channel blocker selective for the N-type calcium channel endothelial dysfunction in the subject is improved.

In some embodiments, after administration of the dual N-type and L-type calcium channel blocker selective for the N-type calcium channel endothelial dysfunction in the subject is improved.

In some embodiments, after administration of the dual N-type and L-type calcium channel blocker selective for the N-type calcium channel oxidative stress in the subject is decreased.

In some embodiments, after administration of the dual N-type and L-type calcium channel blocker selective for the N-type calcium channel oxidative stress in the subject is decreased.

In some embodiments, an antioxidant is not administered to the subject. In some embodiments, the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.

In some embodiments, the occurrence of atrial fibrillation is decreased in the subject. Without wishing to be bound by theory, it is believed that decreasing the occurrence of atrial fibrillation in the subject occurs by means of a decrease in autonomic dysfunction.

In another aspect, disclosed herein is a method of relieving eye pain in a subject, comprising administering a solution of a therapeutically effective amount of a dual N-type and L-type calcium channel blocker selective for the N-type calcium channel to the eye of the subject. A pain-relieving effect of the dual N-type and L-type calcium channel blocker selective for the N-type calcium channel is, without wishing to be bound by theory, believed to be a reduction in intraocular pressure.

In some embodiments, the eye pain is a symptom of a disease or disorder selected from the group consisting of: Sjogren's syndrome, glaucoma, optic neuritis, migraines, conjunctivitis, corneal abrasion, blepharitis, sinusitis, iritis, ocular surgery, corneal abrasions, and Raynaud's syndrome. For example, the eye pain is a symptom of Sjögren's syndrome.

In some embodiments, the method further comprises administering an agent selected from the group consisting of: plaquenil, an antimalarial drug, evoxac, cevimeline, infliximab, or any combination thereof.

In some embodiments, relieving eye pain comprises analgesia of the eye. In some of these embodiments, the subject does not experience anesthesia of the eye.

In another aspect, disclosed herein is a method of treating atrial fibrillation in a subject in need thereof, comprising administering a dual N-type and L-type calcium channel blocker selective for the N-type calcium channel to the subject, wherein after the administration the atrial fibrillation in the subject is improved.

In some embodiments, the method further comprises administering one or more additional therapeutic agents. In some of these embodiments, the one or more additional therapeutic agents include, but are not limited to, soluble guanalate cyclase stimulator, angiotensin receptor blockers (ARBs), angiotensin-converting-enzyme (ACE) inhibitors, selective serotonin reuptake inhibitors (SSRIs), alpha blockers, phosphodiesterase 4 and phosphodiesterase 5 inhibitors, endothelium receptor antagonists, prostaglandins, 5HTA and 5HTB antagonists (sarpogrelate).

In some embodiments, the method further comprises surgery, ablation, cardioversion, weight loss, and physical exercise.

In some embodiments, improving the atrial fibrillation in the subject comprises improvement in the electrical, autonomic, and/or structural remodeling of the heart.

Without wishing to be bound by theory, it is believed that the improvement in atrial fibrillation and reduction in atrial remodeling is a result of reduced norepinephrine, an outcome of N-type calcium channel blockade.

In another aspect, disclosed herein is a method of reducing atrial remodeling in a subject with atrial fibrillation, comprising: administering a dual N-type and L-type calcium channel blocker selective for the N-type calcium channel to the subject.

In another aspect, disclosed herein is a method of improving blood flow in a subject after surgery or revascularization in the subject, comprising administering a dual N-type and L-type calcium channel blocker selective for the N-type calcium channel to the subject. Without wishing to be bound by theory, it is believed that this improvement of blood flow occurs by means of an improvement in sympathetic dysfunction.

In some embodiments, improving blood flow comprises improving arterial and venous blood flow.

In some embodiments, the surgery comprises surgical attachment of tissue to the subject, and wherein after administration blood flow to the surgically attached tissue is improved. In some of these embodiments, the surgically attached tissue is a skin flap.

In another aspect, disclosed herein is a method of improving blood flow in a subject exhibiting reduced blood flow, comprising administering a dual N-type and L-type calcium channel blocker selective for the N-type calcium channel to the subject, wherein after administration blood flow in the subject is improved.

In some embodiments, after administration the temperature of one or more body parts of the subject is increased.

In some embodiments, the subject smokes tobacco regularly.

In another aspect, disclosed herein is a method of treating erectile dysfunction in a subject in need thereof, comprising administering a dual N-type and L-type calcium channel blocker selective for the N-type calcium channel and an agent that treats erectile dysfunction to the subject, wherein after administration erectile dysfunction in the subject is improved.

In some embodiments, the agent that treats erectile dysfunction is selected from sildenafil, tadafenil, and phosphodiesterase type 5 inhibitors.

In some embodiments, after administration of the agent that treats erectile dysfunction to the subject, the blood pressure of the subject is reduced.

In some embodiments, the dual N-type and L-type calcium channel blocker selective for the N-type calcium channel does not include a tetravalent (i.e., quaternized) nitrogen wherein the four substituents bonded to the nitrogen are non-hydrogen substituents.

In some embodiments, the dual N-type and L-type calcium channel blocker selective for the N-type calcium channel does not include a sulfide (i.e., -S-) moiety.

In some embodiments, greater than about 2% (e.g., greater than about 5%, greater than about 10%, greater than about 15%, greater than about 20%, greater than about 25%, greater than about 30%, greater than about 35%, greater than about 40%, greater than about 45%, greater than about 50%, greater than about 55%, greater than about 60%, or greater than about 65%, greater than about 70%, greater than about 75%, greater than about 80%, greater than about 85%, greater than about 90%, greater than about 95%, greater than about 97%, or greater than about 99%) of the dual N-type and L-type calcium channel blocker selective for the N-type calcium channel is anionic at physiological pH (e.g., a pH of from about 7.2 to about 7.6 (e.g., about 7.4)).

In another aspect, disclosed herein is a method of treating a disease or disorder associated with dysregulation of blood flow and sympathetic nervous system overactivity in a subject in need thereof, comprising administering a therapeutically effective amount of a sodium channel blocker to the subject. In certain embodiments, the sodium channel blocker is a Nav 1.7 sodium channel blocker. In some embodiments, the disease or disorder associated with dysregulation of blood flow and sympathetic nervous system overactivity is a disease or disorder associated with sympathetic nervous system overactivity. In some embodiments, the disease or disorder associated with dysregulation of blood flow and sympathetic nervous system overactivity is a disease or disorder characterized by vasoconstriction or neuropathic pain.

In some embodiments, following the administration of the therapeutically effective amount of the Nav 1.7 sodium channel blocker, the subject has an increase in Nav 1.7 inhibition. In some embodiments, the Nav 1.7 inhibition comprises inhibition of the closed state of the Nav 1.7 sodium channel. In some embodiments, the Nav 1.7 inhibition comprises inhibition of the inactivated state of the Nav 1.7 sodium channel. In some embodiments, the inhibition of the closed state of the Nav 1.7 sodium channel is greater than the inhibition of the inactivated state of the Nav 1.7 sodium channel. In some embodiments, the inhibition of the inactivated state of the Nav 1.7 sodium channel is greater than inhibition of the closed state of the Nav 1.7 sodium channel.

In some embodiments, following the administration of the therapeutically effective amount of the Nav 1.7 sodium channel blocker, the subject has an at least 1% increase in Nav 1.7 inhibition. For example, the subject has an at least 5%, at least 10%, at least 20%, at least 30%, at least 40%, at least 50%, at least 60%, at least 70%, at least 80%, at least 90%, or at least 95% increase in Nav 1.7 inhibition. In some embodiments, following the administration of the therapeutically effective amount of the Nav 1.7 sodium channel blocker, the subject has an at least 10% increase in Nav 1.7 inhibition. In some embodiments, following the administration of the therapeutically effective amount of the Nav 1.7 sodium channel blocker, the subject has an at least 20% increase in Nav 1.7 inhibition. In some embodiments, following the administration of the therapeutically effective amount of the Nav 1.7 sodium channel blocker, the subject has an at least 15% increase in Nav 1.7 inhibition. In some embodiments, following the administration of the therapeutically effective amount of the Nav 1.7 sodium channel blocker, the subject has an at least 35% increase in Nav 1.7 inhibition.

In another aspect, disclosed herein is a method of treating a disease or disorder associated with dysregulation of blood flow and sympathetic nervous system overactivity in a subject in need thereof, comprising administering a therapeutically effective amount of a Nav 1.7 sodium channel blocker to the subject.

In some embodiments, the disease or disorder associated with dysregulation of blood flow and sympathetic nervous system overactivity is characterized by neuropathic pain (e.g., chronic neuropathic pain), vasoconstriction, dysesthetic pain, hyperesthetic pain, allodynia, lancinating pain, crampy pain, dull pain, burning pain, body temperature changes of the subject, hyperesthesia, changes in skin or tissue color, edema, changes in skin turgor, rubor, pallor, cyanosis, vasospasm, or any combination thereof. In certain embodiments, the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence. In certain of these embodiments, the disease or disorder is Raynaud's syndrome. For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In another aspect, disclosed herein is a method of treating a disease or disorder characterized by vasoconstriction or neuropathic pain in a subject in need thereof, the method comprising (a) determining that the disease or disorder is associated with vasoconstriction or neuropathic pain; and (b) administering to the subject a therapeutically effective amount of a Nav 1.7 sodium channel blocker. In some embodiments, the disease or disorder is characterized by neuropathic pain.

In some embodiments, the disease or disorder associated with vasoconstriction or neuropathic pain is characterized by neuropathic pain (e.g., chronic neuropathic pain), vasoconstriction, dysesthetic pain, hyperesthetic pain, allodynia, lancinating pain, crampy pain, dull pain, burning pain, body temperature changes of the subject, hyperesthesia, changes in skin or tissue color, edema, changes in skin turgor, rubor, pallor, cyanosis, vasospasm, or any combination thereof. In certain embodiments, the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence. In certain of these embodiments, the disease or disorder is Raynaud's syndrome. For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In some embodiments, the method comprises administering at least one additional therapeutic agent to the subject. The at least one additional therapeutic agent can be administered simultaneously, separately, sequentially, or in combination (e.g., for more than two agents) with the Nav 1.7 sodium channel blocker. Non-limiting examples of additional therapeutic agents include calcium channel blockers, sodium channel blockers (e.g., Nav 1.7 sodium channel blocker), and therapeutic agents that relieve pain.

In certain embodiments, the at least one additional therapeutic agent is selected from the group consisting of: riociguat, amlodipine, nifedipine, nicardipine, conotoxins, cadmium, caroverine, gabapentin, levetiracetam, lamotrigine, NP078585, pregabalin, TROX-1, acetaminophen, non-steroidal anti-inflammatory agents (e.g., ibuprofen), and ziconotide.

In certain embodiments, the at least one additional therapeutic agent is selected from the group consisting of: oxycodone, tramadol, Dilaudid, OxyContin, Cymbalta, a statin, gabapentin, pregabalin, an angiotensin-converting-enzyme (ACE) inhibitor, an angiotensin receptor blocker (ARB), niacin, a proton pump inhibitor, aspirin, Fentanyl Transdermal System, acetaminophen/oxycodone, Roxicodone, Ultram, hydromorphone, Percocet, MS Contin, Butrans, morphine, hydromorphone, methadone, buprenorphine, duloxetine, fentanyl, Duragesic, Endocet, Roxanol, Kadian, Roxicet, ConZip, Methadose, Oxyfast, Dazidox, Fentora, Irenka, Methadone Diskets, Oramorph SR, Roxicodone Intensol, Xtampza ER, Actiq, Belbuca, ETH-Oxydose, Infumorph, naloxone / pentazocine, Oxaydo, Oxydose, OxyIR, ziconotide, Abstral, Astramorph PF, Buprenex, Dolophine, Duramorph, Duramorph PF, Embeda, Lazanda, MorphaBond ER, morphine/naltrexone, Prialt, RMS, Roxanol-T, Sublimaze, Subsys, Talwin Nx, Magnacet, Nalocet, Narvox, Perloxx, Primlev, Xolox, and Prolate.

In another aspect, disclosed herein is a method of treating secondary Raynaud's syndrome in a subject in need thereof, the method comprising administering to the subject (a) a therapeutically effective amount of a Nav 1.7 sodium channel blocker, and (b) at least one additional therapeutic agent.

Without wishing to be bound by theory, it is believed that the secondary Raynaud's syndrome is a consequence of another disease or disorder (also referred to herein as a "primary" disease or disorder). In some embodiments, the primary disease or disorder is lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren'ssyndrome, or any combination thereof. In some embodiments, the at least one additional therapeutic agent is an accepted treatment for the primary disease or disorder.

In certain embodiments, the primary disorder is scleroderma. For example, the primary disorder is scleroderma with interstitial lung disease. In some of embodiments, the method further comprises administering an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the method further comprises administering nintedanib. In some embodiments, the method further comprises administering a calcineurin inhibitor, a non-steroidal anti-inflammatory drug, or both. In some embodiments, the Nav 1.7 sodium channel blocker, the calcineurin inhibitor, and the non-steroidal anti-inflammatory drug are administered separately, sequentially, or simultaneously. In some embodiments, the Nav 1.7 sodium channel blocker, the calcineurin inhibitor, and the non-steroidal anti-inflammatory drug are administered separately. In some embodiments, the Nav 1.7 sodium channel blocker, the calcineurin inhibitor, and the non-steroidal anti-inflammatory drug are administered sequentially. In some embodiments, the Nav 1.7 sodium channel blocker, the calcineurin inhibitor, and the non-steroidal anti-inflammatory drug are administered simultaneously. In some embodiments, the Nav 1.7 sodium channel blocker, the calcineurin inhibitor, and the non-steroidal anti-inflammatory drug are administered simultaneously as a fixed dosage form. In some embodiments, the calcineurin inhibitor is a cyclosporine. In some embodiments, the non steroidal anti-inflammatory drug is aspirin.

In certain embodiments, the primary disorder is lupus. In some embodiments, the method further comprises administering an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the primary disorder is rheumatoid arthritis. In some embodiments, the method further comprises administering an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the primary disorder is Sjögren'ssyndrome. In some embodiments, the method further comprises administering an agent selected from the group consisting of: plaquenil, an antimalarial drug (e.g., hydroxychloroquine), evoxac, cevimeline, infliximab, or any combination thereof.

In another aspect, disclosed herein is a method of reducing a sensation of burning pain, paresthesia, dysesthesia, hypoesthesia, allodynia, or hyperesthesia (e.g., burning pain) in a subject, comprising administering a therapeutically effective amount of a Nav 1.7 sodium channel blocker to a subject. In some embodiments, the sensation of burning pain, paresthesia, dysesthesia, hypoesthesia, allodynia, or hyperesthesia is a symptom of a disease or disorder associated with dysregulation of blood flow and sympathetic nervous system overactivity. In some embodiments, the sensation of burning pain, paresthesia, dysesthesia, hypoesthesia, allodynia, or hyperesthesia in a subject is a symptom of a disease or disorder characterized by neuropathic pain and/or vasoconstriction. A sensation of burning pain can be measured using one or more of the following: the Galer neuropathic pain scale, the ID pain Scale, NPQ, PainDETECT, LANS S, DN4 scales, and/or the Standardized Evaluation of Pain (StEP) tool. See, for example, Cruccu G, Truini A. Tools for assessing neuropathic pain. PLoS Med. 2009;6(4):e1000045. doi: 10. 1371/journal.pmed. 1000045.

In another aspect, disclosed herein is a method of reducing pain or discomfort in a subject caused by a reduction of body temperature in the subject, comprising administering an effective amount of a Nav 1.7 sodium channel blocker to the subject, wherein the reduction of body temperature in the subject is caused by an exposure of the subject to air having a temperature of less than 25°C.

In some embodiments, vasoconstriction in the subject is reduced after administering the Nav 1.7 sodium channel blocker.

In some embodiments, the reduction of body temperature in the subject is caused by an exposure of the subject to air having a temperature of less than 20°C, for example, less than 20 °C, less than 15 °C, less than 10 °C, less than 5 °C, less than 0 °C, less than -5 °C, less than -10 °C, or less than -5 °C.

In some embodiments, the pain or discomfort that is reduced occurs during the exposure of the subject to air having a temperature of less than 25°C.

In some embodiments, the reduction of body temperature in the subject is followed by a restoration to the normal body temperature in the subject, and the pain or discomfort that is reduced occurs after restoration to the normal body temperature in the subject.

In some embodiments, the reduction of body temperature in the subject comprises reduction in the temperature of a region of the body of the subject. In some embodiments, the reduction of body temperature in the subject comprises a reduction in the temperature of a digit, an extremity, or alar circulatory region of the subject. In some embodiments, the reduction of body temperature in the subject comprises reduction in the temperature of a hand or foot of the subject. For example, in some embodiments, the reduction of body temperature in the subject comprises reduction in the temperature of a hand of the subject. For example, the reduction of body temperature in the subject comprises reduction in the temperature of a finger of the subject. In some embodiments, the reduction of body temperature in the subject comprises reduction in the temperature of a foot of the subject.

In another aspect, disclosed herein is a method of reducing susceptibility of a subject to cold-induced pain or discomfort, comprising: administering an effective amount of a Nav 1.7 sodium channel blocker to the subject.

In another aspect, disclosed herein is a method of treating cold-induced pain or discomfort, comprising: administering an effective amount of a Nav 1.7 sodium channel blocker to the subject.

In some embodiments, the subject experiences a lesser degree of the pain or discomfort upon exposure to air having a temperature of less than 25 °C than as compared to a subject that is not administered a Nav 1.7 sodium channel blocker and is exposed to air having a temperature of less than 25 °C. For example, the subject experiences a lesser degree of the pain or discomfort upon exposure to air having a temperature of less than 20 °C than as compared to a subject that is not administered a Nav 1.7 sodium channel blocker and is exposed to air having a temperature of less than 20 °C. For example, the subject experiences a lesser degree of the pain or discomfort upon exposure to air having a temperature of less than 15 °C than as compared to a subject that is not administered a Nav 1.7 sodium channel blocker and is exposed to air having a temperature of less than 15 °C. For example, the subject experiences a lesser degree of the pain or discomfort upon exposure to air having a temperature of less than 10 °C than as compared to a subject that is not administered a Nav 1.7 sodium channel blocker and is exposed to air having a temperature of less than 10 °C. For example, the subject experiences a lesser degree of the pain or discomfort upon exposure to air having a temperature of less than 5 °C than as compared to a subject that is not administered a Nav 1.7 sodium channel blocker and is exposed to air having a temperature of less than 5 °C. For example, the subject experiences a lesser degree of the pain or discomfort upon exposure to air having a temperature of less than 0 °C than as compared to a subject that is not administered a Nav 1.7 sodium channel blocker and is exposed to air having a temperature of less than 0 °C. For example, the subject experiences a lesser degree of the pain or discomfort upon exposure to air having a temperature of less than -10 °C than as compared to a subject that is not administered a Nav 1.7 sodium channel blocker and is exposed to air having a temperature of less than - 10 °C.

In another aspect, disclosed herein is a method of relieving eye pain in a subject, comprising administering a solution of a therapeutically effective amount of a Nav 1.7 sodium channel blocker to the eye of the subject. A pain-relieving effect of the Nav 1.7 sodium channel blocker is, without wishing to be bound by theory, believed to be a reduction in intraocular pressure. Without wishing to be bound by theory, the pain-relieving effect of the Nav 1.7 sodium channel blocker is believed to be inhibition of the Nav 1.7 sodium channel.

In some embodiments, the eye pain is a symptom of a disease or disorder selected from the group consisting of: Sjogren's syndrome, glaucoma, optic neuritis, migraines, conjunctivitis, corneal abrasion, blepharitis, sinusitis, iritis, ocular surgery, corneal abrasions, and Raynaud's syndrome. For example, the eye pain is a symptom of Sjögren's syndrome.

In some embodiments, the method further comprises administering an agent selected from the group consisting of: plaquenil, an antimalarial drug, evoxac, cevimeline, infliximab, or any combination thereof.

In some embodiments, relieving eye pain comprises analgesia of the eye. In some of these embodiments, the subject does not experience anesthesia of the eye.

In some embodiments, the Nav 1.7 sodium channel blocker is ralfinamide, cilnidipine, or a pharmaceutically acceptable salt thereof. In some embodiments, the Nav 1.7 sodium channel blocker is cilnidipine or a pharmaceutically acceptable salt thereof.

In some embodiments, the dosage of the Nav 1.7 sodium channel blocker is about 0.005 mg/kg to about 2 mg/kg. For example, the dosage of the Nav 1.7 sodium channel blocker is about 0.06 mg/kg to about 0.3 mg/kg or about 0.1 mg/kg to about 0.18 mg/kg. In some embodiments, the dosage of the Nav 1.7 sodium channel blocker is from about 5 mg to about 50 mg, for example, from about 5 mg to about 15 mg, from about 15 mg to about 25 mg, from about 5 mg to about 25 mg, about 10 mg, or about 20 mg.

In some embodiments, the method further comprises administering a therapeutically effective amount of an agent that increases blood pressure to the subject. In certain embodiments, the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.

In some embodiments, the blood pressure of the subject before and after administration of the Nav 1.7 sodium channel blocker and the agent that increases blood pressure is substantially the same.

In some embodiments, the Nav 1.7 sodium channel blocker is administered orally, parenterally, transdermally, by inhalation, intranasally, sublingually, neuraxially, or ocularly

In some embodiments, the bone density of the subject does not decrease.

In some embodiments, the method further comprises selecting a subject identified or diagnosed as having reduced bone density for the treatment. In certain embodiments, the subject identified or diagnosed as having reduced bone density is afflicted with osteoporosis. In certain of these embodiments, the subject is female.

In some embodiments, the health or functioning of a kidney in the subject is improved.

In some embodiments, the method further comprises selecting a subject identified or diagnosed as having reduced renal function for the treatment. In certain embodiments, the renal function of the patient is not reduced after treatment. In certain of these embodiments, the renal function of the patient is improved after treatment.

In some embodiments, each administration of the Nav 1.7 sodium channel blocker is separated by at least about 12 hours. For example, each administration of the Nav 1.7 sodium channel blocker is separated by at least about 24 hours, at least about 30 hours, at least about 48 hours, at least about 60 hours, at least about 72 hours, at least about 4 days, at least about 5 days, at least about 6 days, at least about 1 week, at least about 9 days, at least about 12 days, or at least about 2 weeks.

In some embodiments, the Nav 1.7 sodium channel blocker causes sympathetic tone diminution, direct smooth muscle relaxation, or both in the subject.

In some embodiments, after administration of the Nav 1.7 sodium channel blocker nitric oxide is not increased in the subject. This effect is without wishing to be bound by theory, believed to improve, e.g., endothelial function in the subject.

In some embodiments, an antioxidant is not administered to the subject. In certain embodiments, the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.

In some embodiments, the occurrence of atrial fibrillation is decreased in the subject. Without wishing to be bound by theory, it is believed that decreasing the occurrence of atrial fibrillation in the subject occurs by means of a decrease in autonomic dysfunction.

In some embodiments of any method disclosed herein, the subject is not infected with SARS-CoV-2. In some embodiments of any method disclosed herein, the subject is not infected with SARS-CoV-2. In some embodiments of any method disclosed herein, the subject is not being treated for SARS-CoV-2. In some embodiments of any method disclosed herein, the subject is not diagnosed with SARS-CoV-2 (e.g., after the subject is subjected to a test that diagnoses whether the subject is infected with SARS-CoV-2 (e.g., a PCR test, an antigen test, or an antibody test)).

In some embodiments, the Nav 1.7 sodium channel blocker does not include a tetravalent (i.e., quaternized) nitrogen wherein the four substituents bonded to the nitrogen are non-hydrogen substituents.

In some embodiments, the Nav 1.7 sodium channel blocker does not include a sulfide (i.e., -S-) moiety.

In some embodiments, greater than about 2% (e.g., greater than about 5%, greater than about 10%, greater than about 15%, greater than about 20%, greater than about 25%, greater than about 30%, greater than about 35%, greater than about 40%, greater than about 45%, greater than about 50%, greater than about 55%, greater than about 60%, or greater than about 65%, greater than about 70%, greater than about 75%, greater than about 80%, greater than about 85%, greater than about 90%, greater than about 95%, greater than about 97%, or greater than about 99%) of the Nav 1.7 sodium channel blocker is anionic at physiological pH (e.g., a pH of from about 7.2 to about 7.6 (e.g., about 7.4)).

In some embodiments, the disease or disorder that is treated (e.g., the disease or disorder that associated with dysregulation of blood flow and sympathetic nervous system overactivity or the disease or disorder characterized by vasoconstriction or neuropathic pain) is not asthma, rhinitis, conjunctivitis, arthritis, colitis, contact dermatitis, diabetes, eczema, cystitis, gastritis, migraine headache, psoriasis, rosacea, sunburn, pancreatitis, cough, rhinosinusitis, traumatic brain injury, polymicrobial sepsis, a tendinopathy, urticarial, rheumatic disease, inflammation of a blood vessel, gingivitis, or acute lung injury.

In some embodiments, the disease or disorder that is treated (e.g., the disease or disorder that associated with dysregulation of blood flow and sympathetic nervous system overactivity or the disease or disorder characterized by vasoconstriction or neuropathic pain) is not an inflammatory disorder (e.g., a neurogenic inflammatory disorder). In some embodiments, the disease or disorder that is treated (e.g., the disease or disorder that associated with dysregulation of blood flow and sympathetic nervous system overactivity or the disease or disorder characterized by vasoconstriction or neuropathic pain) is not inflammation of a blood vessel. In some embodiments, the disease or disorder that is treated (e.g., the disease or disorder that associated with dysregulation of blood flow and sympathetic nervous system overactivity or the disease or disorder characterized by vasoconstriction or neuropathic pain) is not associated with inflammation of a blood vessel.

### Pharmaceutical Compositions and Formulations

In another aspect, disclosed herein is a pharmaceutical composition comprising a dual N-type and L-type calcium channel blocker selective for the N-type calcium channel, an agent that increases blood pressure, and optionally a pharmaceutically acceptable excipient.

In another aspect, disclosed herein is a pharmaceutical composition comprising a dual N-type and L-type calcium channel blocker selective for the N-type calcium channel; a therapeutic agent selected from the group consisting of: oxycodone, tramadol, Dilaudid, OxyContin, Cymbalta, a statin, gabapentin, pregabalin, an angiotensin-converting-enzyme (ACE) inhibitor, an angiotensin receptor blocker (ARB), niacin, a proton pump inhibitor, aspirin, Fentanyl Transdermal System, acetaminophen/oxycodone, Roxicodone, Ultram, hydromorphone, Percocet, MS Contin, Butrans, morphine, hydromorphone, methadone, buprenorphine, duloxetine, fentanyl, Duragesic, Endocet, Roxanol, Kadian, Roxicet, ConZip, Methadose, Oxyfast, Dazidox, Fentora, Irenka, Methadone Diskets, Oramorph SR, Roxicodone Intensol, Xtampza ER, Actiq, Belbuca, ETH-Oxydose, Infumorph, naloxone / pentazocine, Oxaydo, Oxydose, OxyIR, ziconotide, Abstral, Astramorph PF, Buprenex, Dolophine, Duramorph, Duramorph PF, Embeda, Lazanda, MorphaBond ER, morphine/naltrexone, Prialt, RMS, Roxanol-T, Sublimaze, Subsys, Talwin Nx, Magnacet, Nalocet, Narvox, Perloxx, Primlev, Xolox, and Prolate; and optionally a pharmaceutically acceptable excipient.

In some embodiments, the dual N-type and L-type calcium channel blocker selective for the N-type calcium channel is selected from the group consisting of: cilnidipine, Z-160, zicinotide, ralfinamide, CNV2197944, or pharmaceutically acceptable salts thereof.

In some embodiments, the dual N-type and L-type calcium channel blocker selective for the N-type calcium channel is selected from the group consisting of: cilnidipine, Z-160, ralfinamide, CNV2197944, or pharmaceutically acceptable salts thereof.

In some embodiments, the dual N-type and L-type calcium channel blocker selective for the N-type calcium channel is selected from the group consisting of: cilnidipine, Z-160, CNV2197944, or pharmaceutically acceptable salts thereof.

In some embodiments, the dual N-type and L-type calcium channel blocker selective for the N-type calcium channel is selected from the group consisting of: cilnidipine, Z-160, zicinotide, or pharmaceutically acceptable salts thereof.

In certain embodiments, the dual N-type and L-type calcium channel blocker selective for the N-type calcium channel is cilnidipine or a pharmaceutically acceptable salt thereof.

In some embodiments, the dual N-type and L-type calcium channel blocker selective for the N-type calcium channel is CNV2197944.

In some embodiments, the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.

In another aspect, disclosed herein is a pharmaceutical composition comprising a dual N-type and L-type calcium channel blocker selective for the N-type calcium channel, an agent that treats erectile dysfunction, and optionally a pharmaceutically acceptable excipient.

In some embodiments, the dual N-type and L-type calcium channel blocker selective for the N-type calcium channel is selected from the group consisting of: cilnidipine, Z-160, zicinotide, ralfinamide, CNV2197944, or pharmaceutically acceptable salts thereof.

In some embodiments, the dual N-type and L-type calcium channel blocker selective for the N-type calcium channel is selected from the group consisting of: cilnidipine, Z-160, ralfinamide, CNV2197944, or pharmaceutically acceptable salts thereof.

In some embodiments, the dual N-type and L-type calcium channel blocker selective for the N-type calcium channel is selected from the group consisting of: cilnidipine, Z-160, CNV2197944, or pharmaceutically acceptable salts thereof.

In some embodiments, the dual N-type and L-type calcium channel blocker selective for the N-type calcium channel is selected from the group consisting of: cilnidipine, Z-160, zicinotide, or pharmaceutically acceptable salts thereof.

In certain embodiments, the dual N-type and L-type calcium channel blocker selective for the N-type calcium channel is cilnidipine or a pharmaceutically acceptable salt thereof.

In some embodiments, the dual N-type and L-type calcium channel blocker selective for the N-type calcium channel is CNV2197944.

In some embodiments, the agent that treats erectile dysfunction is sildenafil, tadafenil, or phosphodiesterase type 5 inhibitors.

In another aspect, disclosed herein is a pharmaceutical composition comprising a dual N-type and L-type calcium channel blocker selective for the N-type calcium channel; cyclophosphamide, nintedanib, methotrexate, mycophenolate, a glucocorticoid (e.g., prednisone, dexamethasone, and hydrocortisone), a non steroidal anti-inflammatory drug

(e.g., aspirin, ibuprofen, or naproxen), D-penicillamine, a diuretic, omeprazole, bosentan, epoprostenol, enalapril, Lisinopril, captopril, or any combination thereof; and optionally a pharmaceutically acceptable excipient.

In another aspect, disclosed herein is a pharmaceutical composition comprising a dual N-type and L-type calcium channel blocker selective for the N-type calcium channel; nintedanib; and optionally a pharmaceutically acceptable excipient.

In another aspect, disclosed herein is a pharmaceutical composition comprising a dual N-type and L-type calcium channel blocker selective for the N-type calcium channel; an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof; and optionally a pharmaceutically acceptable excipient.

In another aspect, disclosed herein is a pharmaceutical composition comprising a dual N-type and L-type calcium channel blocker selective for the N-type calcium channel; disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof; and optionally a pharmaceutically acceptable excipient.

In another aspect, disclosed herein is a pharmaceutical composition comprising a dual N-type and L-type calcium channel blocker selective for the N-type calcium channel; plaquenil, an antimalarial drug (e.g., hydroxychloroquine), evoxac, cevimeline, infliximab, or any combination thereof; and optionally a pharmaceutically acceptable excipient.

In another aspect, disclosed herein is a pharmaceutical composition comprising a dual N-type and L-type calcium channel blocker selective for the N-type calcium channel; nintedanib, pirfenidone, or any combination thereof; and optionally a pharmaceutically acceptable excipient.

In another aspect, disclosed herein is a pharmaceutical composition comprising a dual N-type and L-type calcium channel blocker selective for the N-type calcium channel, a calcineurin inhibitor, and optionally a pharmaceutically acceptable excipient.

In some embodiments, the calcineurin inhibitor is a cyclosporine.

In some embodiments, the pharmaceutical composition further comprises a non-steroidal anti-inflammatory drug. In some embodiments, the non steroidal anti-inflammatory drug is aspirin.

In some embodiments, the dual N-type and L-type calcium channel blocker selective for the N-type calcium channel is selected from the group consisting of: cilnidipine, Z-160, zicinotide, ralfinamide, CNV2197944, or pharmaceutically acceptable salts thereof. In some embodiments, the dual N-type and L-type calcium channel blocker selective for the N-type calcium channel is selected from the group consisting of: cilnidipine, Z-160, ralfinamide, CNV2197944, or pharmaceutically acceptable salts thereof. In some embodiments, the dual N-type and L-type calcium channel blocker selective for the N-type calcium channel is selected from the group consisting of: cilnidipine, Z-160, CNV2197944, or pharmaceutically acceptable salts thereof. In some embodiments, the dual N-type and L-type calcium channel blocker selective for the N-type calcium channel is selected from the group consisting of: cilnidipine, Z-160, or pharmaceutically acceptable salts thereof. In certain embodiments, the dual N-type and L-type calcium channel blocker selective for the N-type calcium channel is a dihydropyridine N-type calcium channel blocker. In certain of these embodiments, the dual N-type and L-type calcium channel blocker selective for the N-type calcium channel is cilnidipine or a pharmaceutically acceptable salt thereof. In some embodiments, the dual N-type and L-type calcium channel blocker selective for the N-type calcium channel is CNV2197944.

In some embodiments, a chemical entity (e.g., the dual N-type and L-type calcium channel blocker selective for the N-type calcium channel and/or sodium channel blocker (e.g., Nav 1.7 sodium channel blocker)) is administered as a pharmaceutical composition that includes the dual N-type and L-type calcium channel blocker selective for the N-type calcium channel and/or sodium channel blocker (e.g., Nav 1.7 sodium channel blocker) and one or more pharmaceutically acceptable excipients, and optionally one or more additional therapeutic agents as described herein.

In some embodiments, the dual N-type and L-type calcium channel blocker selective for the N-type calcium channel and/or sodium channel blocker (e.g., Nav 1.7 sodium channel blocker) can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-β-cyclodextrins, or other solubilized derivatives can also be used to enhance delivery of compounds described herein. Dosage forms or compositions containing a chemical entity as described herein in the range of 0.005% to 100% with the balance made up from nontoxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

### Routes of Administration and Composition Components

In some embodiments, the chemical entities described herein or a pharmaceutical composition thereof can be administered to subject in need thereof by any accepted route of administration. Acceptable routes of administration include, but are not limited to, oral, parenteral, transdermal, intranasal, sublingual, neuraxial, or ocular .

Compositions can be formulated for parenteral administration, e.g., formulated for injection via the intravenous, intramuscular, sub-cutaneous, or even intraperitoneal routes. Typically, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

In other embodiments, the compounds described herein or a pharmaceutical composition thereof are suitable for local delivery to the digestive or GI tract by way of oral administration (e.g., solid or liquid dosage forms.).

Solid dosage forms for oral administration include capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a chemical entity provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils, PEG's, poloxamer 124 or triglycerides) is encapsulated in a capsule (gelatin or cellulose base capsule). Unit dosage forms in which one or more chemical entities provided herein or additional active agents are physically separated are also contemplated; e.g., capsules with granules (or tablets in a capsule) of each drug; two-layer tablets; two-compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid.

In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the chemical entity to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802, which is incorporated herein by reference in its entirety.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap.

Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, ETDA, SofZia (boric acid, propylene glycol, sorbitol, and zinc chloride; Alcon Laboratories, Inc.), Purite (stabilized oxychloro complex; Allergan, Inc.)).

Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non-sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, each administration of the dual N-type and L-type calcium channel blocker selective for the N-type calcium channel and/or sodium channel blocker (e.g., Nav 1.7 sodium channel blocker) is separated by at least about 12 hours. For example, each administration of the dual N-type and L-type calcium channel blocker selective for the N-type calcium channel is separated by at least about 24 hours, at least about 30 hours, at least about 48 hours, at least about 60 hours, at least about 72 hours, at least about 4 days, at least about 5 days, at least about 6 days, at least about 1 week, at least about 9 days, at least about 12 days, or at least about 2 weeks.

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

### Exemplary Embodiments

1. A method of treating a disease or disorder associated with dysregulation of blood flow and sympathetic nervous system overactivity in a subject in need thereof, comprising administering a therapeutically effective amount of a dual N-type and L-type calcium channel blocker selective for the N-type calcium channel to the subj ect.
2. The method of embodiment 1, wherein one or more side effects experienced by the subject after administration of the dual N-type and L-type calcium channel blocker selective for the N-type calcium channel are less severe or less frequent than as compared to the side effects experienced by a subject after administration of a therapeutically effective amount of a non-N-selective calcium channel blocker useful to treat the disease or disorder.
3. The method of any one of embodiments 1-2, wherein the disease or disorder associated with dysregulation of blood flow and sympathetic nervous system overactivity is characterized by neuropathic pain, vasoconstriction, dysesthetic pain, hyperesthetic pain, allodynia, lancinating pain, crampy pain, dull pain, burning pain, body temperature changes of the subject, changes in skin or tissue color, edema, changes in skin turgor, rubor, pallor, cyanosis, vasospasm, or any combination thereof.
4. The method of any one of embodiments 1-3, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
5. The method of any one of embodiments 1-4, wherein the disease or disorder is Raynaud's syndrome.
6. The method of any one of embodiments 4-5, wherein Raynaud's syndrome is selected from the group consisting of: primary Raynaud's syndrome; secondary Raynaud's syndrome; Raynaud's syndrome of the nipple, nose, ear, penis, tongue, and/or any alar circulatory region.
7. The method of embodiment 6, wherein the Raynaud's syndrome is secondary Raynaud's syndrome.
8. The method of any one of embodiments 2-7, wherein the non-N-selective calcium channel blocker is selected from the group consisting of: nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine, nitrendipine, and pharmaceutically acceptable salts thereof.
9. The method of any one of embodiments 2-8, wherein the side effects are selected from the group consisting of: constipation, nausea, headache, fatigue, rash, edema, pulmonary edema, peripheral edema, heart rate changes, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, itching, and combinations thereof.
10. The method of any one of embodiments 5-7, wherein the subject is also diagnosed with hypertension; and wherein after administration of the dual N-type and L-type calcium channel blocker selective for the N-type calcium channel to the subject, the blood pressure of the subject is reduced.
11. The method of embodiment 10, wherein the systolic blood pressure of the subject is reduced by greater than 10 mm Hg.
12. The method of embodiment 7, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof.
13. The method of any one of embodiments 7 and 12, wherein the subject is being treated for scleroderma.
14. The method of any one of embodiments 7 and 12, wherein the subject is being treated for scleroderma with interstitial lung disease.
15. The method of any one of embodiments 13-14, further comprising administering an agent selected from the group consisting of: a calcineurin inhibitor, cyclophosphamide, nintedanib, methotrexate, mycophenolate, a glucocorticoid, a non steroidal anti-inflammatory drug, D-penicillamine, a diuretic, omeprazole, bosentan, epoprostenol, enalapril, lisinopril, captopril, or any combination thereof.
16. The method of embodiment 15, further comprising administering nintedanib.
17. The method of embodiment 15, further comprising administering a calcineurin inhibitor, a non-steroidal anti-inflammatory drug, or both.
18. The method of embodiment 17, wherein the dual N-type and L-type calcium channel blocker selective for the N-type calcium channel, the calcineurin inhibitor, and the non-steroidal anti-inflammatory drug are administered separately, sequentially, or simultaneously.
19. The method of embodiment 18, wherein the dual N-type and L-type calcium channel blocker selective for the N-type calcium channel, the calcineurin inhibitor, and the non-steroidal anti-inflammatory drug are administered simultaneously.
20. The method of embodiment 19, wherein the dual N-type and L-type calcium channel blocker selective for the N-type calcium channel, the calcineurin inhibitor, and the non-steroidal anti-inflammatory drug are administered simultaneously as a fixed dosage form.
21. The method of any one of embodiments 15 and 17-20, wherein the calcineurin inhibitor is a cyclosporine.
22. The method of any one of embodiments 15 and 17-20, wherein the non steroidal anti-inflammatory drug is aspirin.
23. The method of any one of embodiments 7 and 12, wherein the subject is being treated for lupus.
24. The method of embodiment 23, further comprising administering an agent selected from the group consisting of: an antimalarial drug, a non-steroidal anti-inflammatory drug, belimumab, a corticosteroid, an immunosuppressant, or any combination thereof.
25. The method of any one of embodiments 7 and 12, wherein the subject is being treated for rheumatoid arthritis.
26. The method of embodiment 25, further comprising administering an agent selected from the group consisting of: methotrexate, sulfasalazine, a non-steroidal anti-inflammatory drug, a corticosteroid, a biologic, or any combination thereof.
27. The method of any one of embodiments 7 and 12, wherein the subject is being treated for Sjögren's syndrome.
28. The method of embodiment 27, further comprising administering an agent selected from the group consisting of: plaquenil, an antimalarial drug, evoxac, cevimeline, infliximab, or any combination thereof.
29. The method of any one of embodiments 7 and 12, wherein the subject is being treated for idiopathic pulmonary fibrosis.
30. The method of embodiment 29, further comprising administering an agent selected from the group consisting of: nintedanib, pirfenidone, or any combination thereof.
31. A method of treating a disease or disorder characterized by vasoconstriction or neuropathic pain in a subject in need thereof, the method comprising (a) determining that the disease or disorder is associated with vasoconstriction or neuropathic pain; and (b) administering to the subject a therapeutically effective amount of a dual N-type and L-type calcium channel blocker selective for the N-type calcium channel.
32. The method of embodiment 31, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
33. The method of any one of embodiments 31-32, wherein the disease or disorder is Raynaud's syndrome.
34. The method of any one of embodiments 32-33, wherein Raynaud's syndrome is selected from the group consisting of: primary Raynaud's syndrome; secondary Raynaud's syndrome; Raynaud's syndrome of the nipple, nose, ear, penis, tongue, and/or any alar circulatory region.
35. The method of embodiment 34, wherein the Raynaud's syndrome is secondary Raynaud's syndrome.
36. The method of any one of embodiments 33-35, wherein the subject is also diagnosed with hypertension; and wherein after administration of the dual N-type and L-type calcium channel blocker selective for the N-type calcium channel to the subject, the blood pressure of the subject is reduced.
37. The method of embodiment 36, wherein the systolic blood pressure of the subject is reduced by greater than 10 Hg.
38. The method of embodiment 35, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof.
39. The method of embodiment 38, wherein the subject is being treated for scleroderma.
40. The method of embodiment 38, wherein the subject is being treated for scleroderma with interstitial lung disease.
41. The method of any one of embodiments 39-40, further comprising administering an agent selected from the group consisting of: a calcineurin inhibitor, cyclophosphamide, nintedanib, methotrexate, mycophenolate, a glucocorticoid, a non steroidal anti-inflammatory drug, D-penicillamine, a diuretic, omeprazole, bosentan, epoprostenol, enalapril, Lisinopril, captopril, or any combination thereof.
42. The method of any one of embodiments 39-41, further comprising administering nintedanib.
43. The method of any one of embodiments 41-42, further comprising administering a calcineurin inhibitor, a non-steroidal anti-inflammatory drug, or both.
44. The method of embodiment 43, wherein the dual N-type and L-type calcium channel blocker selective for the N-type calcium channel, the calcineurin inhibitor, and the non-steroidal anti-inflammatory drug are administered separately, sequentially, or simultaneously.
45. The method of embodiment 44, wherein the dual N-type and L-type calcium channel blocker selective for the N-type calcium channel, the calcineurin inhibitor, and the non-steroidal anti-inflammatory drug are administered simultaneously.
46. The method of embodiment 45, wherein the dual N-type and L-type calcium channel blocker selective for the N-type calcium channel, the calcineurin inhibitor, and the non-steroidal anti-inflammatory drug are administered simultaneously as a fixed dosage form.
47. The method of any one of embodiments 41 and 43-46, wherein the calcineurin inhibitor is a cyclosporine.
48. The method of any one of embodiments 41 and 43-47, wherein the non steroidal anti-inflammatory drug is aspirin.
49. The method of embodiment 38, wherein the subject is being treated for lupus.
50. The method of embodiment 49, further comprising administering an agent selected from the group consisting of: an antimalarial drug, a non-steroidal anti-inflammatory drug, belimumab, a corticosteroid, an immunosuppressant, or any combination thereof.
51. The method of embodiment 38, wherein the subject is being treated for rheumatoid arthritis.
52. The method of embodiment 51, further comprising administering an agent selected from the group consisting of: methotrexate, sulfasalazine, a non-steroidal anti-inflammatory drug, a corticosteroid, a biologic, or any combination thereof.
53. The method of embodiment 38, wherein the subject is being treated for Sjögren's syndrome.
54. The method of embodiment 53, further comprising administering an agent selected from the group consisting of: plaquenil, an antimalarial drug, evoxac, cevimeline, infliximab, or any combination thereof.
55. The method of embodiment 38, wherein the subject is being treated for idiopathic pulmonary fibrosis.
56. The method of embodiment 55, further comprising administering an agent selected from the group consisting of: nintedanib, pirfenidone, or any combination thereof.
57. The method of any one of embodiments 31-56, comprising administering at least one additional calcium channel blocker to the subject.
58. The method of embodiment 57, wherein the at least one additional calcium channel blocker is selected from the group consisting of: amlodipine, nifedipine, nicardipine, nimodipine, verapamil, diltiazem, felodipine, isradipine, nisoldipine, and nitrendipine.
59. A method of reducing a sensation of burning pain, paresthesia, dysesthesia, hypoesthesia, allodynia, or hyperesthesia in a subject in need thereof, comprising administering a therapeutically effective amount of a dual N-type and L-type calcium channel blocker selective for the N-type calcium channel to a subject.
60. The method of any one of embodiments 1-59, further comprising administering to the subject a therapeutically effective amount of an agent that increases blood pressure.
61. The method of embodiment 60, wherein the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.
62. The method of embodiment 61, wherein the blood pressure of the subject before and after administration of the dual N-type and L-type calcium channel blocker selective for the N-type calcium channel and the agent that increases blood pressure is substantially the same.
63. The method of any one of embodiments 1-62, wherein the bone density of the subject does not decrease.
64. The method of any one of embodiments 1-62, further comprising selecting a subject identified or diagnosed as having reduced bone density for the treatment.
65. The method of embodiment 64, wherein the subject identified or diagnosed as having reduced bone density is afflicted with osteoporosis.
66. The method of any one of embodiments 64-65, wherein the subject is female.
67. The method of any one of embodiments 1-66, further comprising selecting a subject identified or diagnosed as having reduced renal function for the treatment.
68. The method of embodiment 67, wherein the renal function of the patient is not reduced after treatment.
69. A method of reducing pain or discomfort in a subject in need thereof caused by a reduction of body temperature in the subject, comprising administering an effective amount of a dual N-type and L-type calcium channel blocker selective for the N-type calcium channel to the subject, wherein the reduction of body temperature in the subject is caused by an exposure of the subject to air having a temperature of less than 25°C.
70. The method of embodiment 69, wherein vasoconstriction in the subject is reduced after administering the dual N-type and L-type calcium channel blocker selective for the N-type calcium channel.
71. The method of any one of embodiments 69-70, wherein the reduction of body temperature in the subject is caused by an exposure of the subject to air having a temperature of less than 10°C.
72. The method of any one of embodiments 69-71, wherein the reduction of body temperature in the subject comprises reduction in the temperature of a digit, an extremity, or alar circulatory region of the subject.
73. The method of any one of embodiments 69-72, wherein the reduction of body temperature in the subject comprises reduction in the temperature of a hand or a foot of the subject.
74. A method of reducing susceptibility of a subject to cold-induced pain or discomfort, comprising: administering an effective amount of a dual N-type and L-type calcium channel blocker selective for the N-type calcium channel to the subj ect.
75. A method of treating cold-induced pain or discomfort in a subject, comprising: administering an effective amount of a dual N-type and L-type calcium channel blocker selective for the N-type calcium channel to the subject.
76. The method of any one of embodiments 74-75, wherein the subject experiences a lesser degree of the pain or discomfort upon exposure to air having a temperature of less than 25 °C than as compared to a subject that is not administered a dual N-type and L-type calcium channel blocker selective for the N-type calcium channel and is exposed to air having a temperature of less than 25 °C.
77. A method of treating sickle cell disease in a subject in need thereof, comprising administering an effective amount of a dual N-type and L-type calcium channel blocker selective for the N-type calcium channel to the subject.
78. The method of embodiment 77, wherein the sickle cell disease is associated with an SS genotype.
79. The method of embodiment 77, wherein the sickle cell disease is associated with an SC genotype.
80. The method of any one of embodiments 77-79, wherein the treating comprises decreasing the severity of one or more symptoms.
81. The method of any one of embodiments 77-80, wherein the treating comprises decreasing the frequency of one or more symptoms.
82. The method of any one of embodiments 77-81, wherein the treating comprises decreasing the duration of one or more symptoms.
83. The method of any one of embodiments 77-82, wherein the one or more symptoms includes pain.
84. The method of any one of embodiments 77-83, wherein the method further comprises preventing or reducing the severity of one or more diseases or disorders associated with sickle cell disease selected from the group consisting of: bacterial infections, ischemic stroke, hemorrhagic stroke, silent stroke, cholelithiasis, cholecystitis, deterioration of the bones, hyposplenism, priapism, osteomyelitis, acute papillary necrosis, leg ulcers, an eye disorder, pulmonary hypertension, and a kidney disorder.
85. The method of embodiment 84, wherein the deterioration of the bones comprises avascular necrosis.
86. The method of embodiment 84, wherein the eye disorder is selected from the group consisting of: background retinopathy, proliferative retinopathy, vitreous haemorrhages, and retinal detachments.
87. The method of embodiment 84, wherein the kidney disorder is chronic kidney failure.
88. The method of any one of embodiments 77-87, wherein the method further comprises administering a second therapeutic agent selected from the group consisting of: hydroxyurea, L-glutamine, crizanlizumab, voxelotor, and senicapoc.
89. A method of treating vasculitis in a subject in need thereof, comprising administering an effective amount of a dual N-type and L-type calcium channel blocker selective for the N-type calcium channel to the subject.
90. The method of embodiment 89, wherein the vasculitis is selected from large vessel vasculitis, medium vessel vasculitis, and small vessel vasculitis.
91. The method of embodiment 90, wherein the vasculitis is large vessel vasculitis.
92. The method of embodiment 91, wherein the large vessel vasculitis is Takayasu arteritis.
93. The method of embodiment 90, wherein the vasculitis is medium vessel vasculitis.
94. The method of embodiment 93, wherein the medium vessel vasculitis is Buerger's disease or polyarteritis nodosa.
95. The method of embodiment 90, wherein the vasculitis is small vessel vasculitis.
96. The method of embodiment 95, wherein the small vessel vasculitis is selected from granulomatosis with polyangiitis, Churg-Strauss syndrome, and microscopic polyangiitis.
97. A method of treating thrombosis in a subject in need thereof, comprising administering an effective amount of a dual N-type and L-type calcium channel blocker selective for the N-type calcium channel to the subject.
98. The method of embodiment 97, wherein the thrombosis is venous thrombosis or arterial thrombosis.
99. The method of embodiment 97, wherein the thrombosis is selected from deep vein thrombosis, portal vein thrombosis, renal vein thrombosis, jugular vein thrombosis, Budd-Chiari syndrome, Paget-Schroetter disease, cerebral venous sinus thrombosis, thrombotic stroke, and myocardial infarction.
100. A method of treating a kidney disorder in a subject in need thereof, comprising administering an effective amount of a dual N-type and L-type calcium channel blocker selective for the N-type calcium channel to the subject, wherein the kidney disorder is a complication of a disease or disorder.
101. The method of embodiment 100, wherein the kidney disorder is selected from chronic kidney disease, kidney stones, glomerulonephritis, polycystic kidney disease, and urinary tract infection.
102. A method of treating hypertension and chronic kidney disease in a subject in need thereof, comprising administering an effective amount of a dual N-type and L-type calcium channel blocker selective for the N-type calcium channel to the subject.
103. The method of embodiment 102, wherein the subject is undergoing dialysis support, and wherein the subject requires less dialysis support than if the subject were not administered an effective amount of a dual N-type and L-type calcium channel blocker selective for the N-type calcium channel.
104. The method of any one of embodiments 102-103, wherein the method further comprises administering a second therapeutic agent selected from the group consisting of: azilsartan, candesartan, eprosartan, atorvastatin, fluvastatin, lovastatin, pravastatin, simvastatin, rosuvastatin, erythropoietin chlorothiazide, chlorthalidone, bumetanide, vitamin D, or calcium supplements.
105. A method of relieving eye pain in a subject in need thereof, comprising administering a solution of a therapeutically effective amount of a dual N-type and L-type calcium channel blocker selective for the N-type calcium channel to the eye of the subject.
106. The method of embodiment 105, wherein the eye pain is a symptom of a disease or disorder selected from the group consisting of: Sjögren's syndrome, glaucoma, optic neuritis, migraines, conjunctivitis, corneal abrasion, blepharitis, sinusitis, iritis, ocular surgery, corneal abrasions, and Raynaud's syndrome.
107. The method of embodiment 106, wherein the eye pain is a symptom of Sjögren'ssyndrome.
108. The method of embodiment 107, further comprising administering an agent selected from the group consisting of: plaquenil, an antimalarial drug, evoxac, cevimeline, infliximab, or any combination thereof.
109. The method of any one of embodiments 105-108, wherein the subject does not experience anesthesia of the eye.
110. A method of treating atrial fibrillation in a subject in need thereof, comprising administering a dual N-type and L-type calcium channel blocker selective for the N-type calcium channel to the subject, wherein after the administration the atrial fibrillation in the subject is improved.
111. A method of reducing atrial remodeling in a subject with atrial fibrillation, comprising: administering a dual N-type and L-type calcium channel blocker selective for the N-type calcium channel to the subject.
112. A method of improving blood flow in a subject after surgery or revascularization in the subject, comprising administering a dual N-type and L-type calcium channel blocker selective for the N-type calcium channel to the subject.
113. The method of embodiment 112, wherein the surgery comprises surgical attachment of tissue to the subject, and wherein after administration blood flow to the surgically attached tissue is improved.
114. The method of embodiment 113 wherein the surgically attached tissue is a skin flap.
115. A method of improving blood flow in a subject exhibiting reduced blood flow, comprising administering a dual N-type and L-type calcium channel blocker selective for the N-type calcium channel to the subject, wherein after administration blood flow in the subject is improved.
116. The method of embodiment 115, wherein after administration the temperature of one or more body parts of the subject is increased.
117. The method of any one of embodiments 115-116, wherein the subject smokes tobacco regularly.
118. A method of treating erectile dysfunction in a subject in need thereof, comprising administering a dual N-type and L-type calcium channel blocker selective for the N-type calcium channel and an agent that treats erectile dysfunction to the subject, wherein after administration erectile dysfunction in the subject is improved.
119. The method of embodiment 118, wherein the agent that treats erectile dysfunction is selected from the group consisting of: sildenafil, tadafenil, and phosphodiesterase type 5 inhibitors.
120. The method of any one of embodiments 1-119, wherein the dual N-type and L-type calcium channel blocker selective for the N-type calcium channel is selected from the group consisting of: cilnidipine, Z-160, CNV2197944, or pharmaceutically acceptable salts thereof.
121. The method of any one of embodiments 1-120, wherein the dual N-type and L-type calcium channel blocker selective for the N-type calcium channel is cilnidipine or a pharmaceutically acceptable salt thereof.
122. The method of any one of embodiments 1-121, wherein the dosage of the dual N-type and L-type calcium channel blocker selective for the N-type calcium channel is about 0.005 mg/kg to about 2 mg/kg.
123. The method of any one of embodiments 1-121, wherein the dosage of the dual N-type and L-type calcium channel blocker selective for the N-type calcium channel is about 0.06 mg/kg to about 0.3 mg/kg.
124. The method of any one of embodiments 1-121, wherein the dosage of the dual N-type and L-type calcium channel blocker selective for the N-type calcium channel is about 0.1 mg/kg to about 0.18 mg/kg.
125. The method of any one of embodiments 1-124, wherein the dual N-type and L-type calcium channel blocker selective for the N-type calcium channel is administered orally, parenterally, transdermally, by inhalation, intranasally, sublingually, neuraxially, or ocularly.
126. The method of any one of embodiments 1-125, wherein each administration of the dual N-type and L-type calcium channel blocker selective for the N-type calcium channel is separated by at least about 24 hours.
127. The method of any one of embodiments 1-125, wherein each administration of the dual N-type and L-type calcium channel blocker selective for the N-type calcium channel is separated by at least about 48 hours.
128. The method of any one of embodiments 1-125, wherein each administration of the dual N-type and L-type calcium channel blocker selective for the N-type calcium channel is separated by at least about 72 hours.
129. The method of any one of embodiments 1-125, wherein each administration of the dual N-type and L-type calcium channel blocker selective for the N-type calcium channel is separated by at least about 1 week.
130. The method of any one of embodiments 1-129, wherein the dual N-type and L-type calcium channel blocker selective for the N-type calcium channel causes sympathetic tone diminution, direct smooth muscle relaxation, or both in the subj ect.
131. The method of any one of embodiments 1-130, wherein the dual N-type and L-type calcium channel blocker selective for the N-type calcium channel exhibits at least a 50-fold selectivity for the N-type calcium channel over an L-type calcium channel.
132. The method of any one of embodiments 1-130, wherein the dual N-type and L-type calcium channel blocker selective for the N-type calcium channel exhibits a 50-fold to 100-fold selectivity for the N-type calcium channel over an L-type calcium channel.
133. The method of any one of embodiments 1-132, wherein the dual N-type and L-type calcium channel blocker selective for the N-type calcium channel further inhibits a Nav 1.7 sodium channel.
134. The method of any one of embodiments 1-133, wherein after administration of the dual N-type and L-type calcium channel blocker selective for the N-type calcium channel nitric oxide is not increased in the subject.
135. The method of any one of embodiments 1-134, wherein after administration of the dual N-type and L-type calcium channel blocker selective for the N-type calcium channel endothelial dysfunction in the subject is improved.
136. The method of any one of embodiments 1-135, wherein after administration of the dual N-type and L-type calcium channel blocker selective for the N-type calcium channel oxidative stress in the subject is decreased.
137. The method of any one of embodiments 1-136, wherein an antioxidant is not administered to the subject.
138. The method of embodiment 137, wherein the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.
139. A method of treating a disease or disorder associated with dysregulation of blood flow and sympathetic nervous system overactivity in a subject in need thereof, comprising administering a therapeutically effective amount of a Nav 1.7 sodium channel blocker to the subject.
140. The method of embodiment 139, wherein the disease or disorder associated with dysregulation of blood flow and sympathetic nervous system overactivity is characterized by neuropathic pain, vasoconstriction, dysesthetic pain, hyperesthetic pain, allodynia, lancinating pain, crampy pain, dull pain, burning pain, body temperature changes of the subject, changes in skin or tissue color, edema, changes in skin turgor, rubor, pallor, cyanosis, vasospasm, or any combination thereof.
141. The method of any one of embodiments 139-140, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
142. The method of any one of embodiments 139-141, wherein the disease or disorder is Raynaud's syndrome.
143. The method of embodiment 142, wherein Raynaud's syndrome is selected from the group consisting of: primary Raynaud's syndrome; secondary Raynaud's syndrome; Raynaud's syndrome of the nipple, nose, ear, penis, tongue, and/or any alar circulatory region.
144. The method of any one of embodiments 139-143, wherein the Nav 1.7 sodium channel blocker is cilnidipine or a pharmaceutically acceptable salt thereof.
145. The method of any one of embodiments 139-144, wherein the dosage of the Nav 1.7 sodium channel blocker is about 0.005 mg/kg to about 2 mg/kg.
146. The method of any one of embodiments 139-144, wherein the dosage of the Nav 1.7 sodium channel blocker is about 0.06 mg/kg to about 0.3 mg/kg.
147. The method of any one of embodiments 139-144, wherein the dosage of the Nav 1.7 sodium channel blocker is about 0.1 mg/kg to about 0.18 mg/kg.
148. The method of any one of embodiments 139-147, further comprising administering a therapeutically effective amount of an agent that increases blood pressure to the subject.
149. The method of embodiment 148, wherein the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.
150. The method of any one of embodiments 148-149, wherein the blood pressure of the subject before and after administration of the Nav 1.7 sodium channel blocker and the agent that increases blood pressure is substantially the same.
151. The method of any one of embodiments 139-150, wherein the Nav 1.7 sodium channel blocker is administered orally, parenterally, transdermally, by inhalation, intranasally, sublingually, neuraxially, or ocularly.
152. The method of any one of embodiments 139-151, wherein the bone density of the subject does not decrease.
153. The method of any one of embodiments 139-152, further comprising selecting a subject identified or diagnosed as having reduced bone density for the treatment.
154. The method of embodiment 153, wherein the subject identified or diagnosed as having reduced bone density is afflicted with osteoporosis.
155. The method of embodiment 154, wherein the subject is female.
156. The method of any one of embodiments 139-155, wherein the health or functioning of a kidney in the subject is improved.
157. The method of any one of embodiments 139-156, further comprising selecting a subject identified or diagnosed as having reduced renal function for the treatment.
158. The method of embodiment 157, wherein the renal function of the patient is not reduced after treatment.
159. The method of embodiment 158, wherein the renal function of the patient is improved after treatment.
160. The method of any one of embodiments 139-159, wherein each administration of the Nav 1.7 sodium channel blocker is separated by at least about 24 hours.
161. The method of any one of embodiments 139-159, wherein each administration of the Nav 1.7 sodium channel blocker is separated by at least about 48 hours.
162. The method of any one of embodiments 139-159, wherein each administration of the Nav 1.7 sodium channel blocker is separated by at least about 72 hours.
163. The method of any one of embodiments 139-159, wherein each administration of the Nav 1.7 sodium channel blocker is separated by at least about 1 week.
164. The method of any one of embodiments 139-163, wherein the Nav 1.7 sodium channel blocker causes sympathetic tone diminution, direct smooth muscle relaxation, or both in the subject.
165. The method of any one of embodiments 139-164, wherein after administration of the Nav 1.7 sodium channel blocker nitric oxide is not increased in the subject.
166. The method of any one of embodiments 139-165, wherein an antioxidant is not administered to the subject.
167. The method of embodiment 166, wherein the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.
168. A pharmaceutical composition comprising a dual N-type and L-type calcium channel blocker selective for the N-type calcium channel, an agent that increases blood pressure, and optionally a pharmaceutically acceptable excipient.
169. The composition of embodiment 168, wherein the dual N-type and L-type calcium channel blocker selective for the N-type calcium channel is selected from the group consisting of: cilnidipine, Z-160, CNV2197944, or pharmaceutically acceptable salts thereof.
170. The composition of any one of embodiments 168-169, wherein the dual N-type and L-type calcium channel blocker selective for the N-type calcium channel is cilnidipine or a pharmaceutically acceptable salt thereof.
171. The composition of any one of embodiments 168-170, wherein the agent that increases blood pressure is selected from the group consisting of: midodrine, cortisone, prednisone, trimipramine, and venlafaxine.
172. A pharmaceutical composition comprising a dual N-type and L-type calcium channel blocker selective for the N-type calcium channel, an agent that treats erectile dysfunction, and optionally a pharmaceutically acceptable excipient.
173. The composition of embodiment 172, wherein the dual N-type and L-type calcium channel blocker selective for the N-type calcium channel is selected from the group consisting of: cilnidipine, Z-160, CNV2197944, or pharmaceutically acceptable salts thereof.
174. The composition of any one of embodiments 172-173, wherein the dual N-type and L-type calcium channel blocker selective for the N-type calcium channel is cilnidipine or a pharmaceutically acceptable salt thereof.
175. The composition of any one of embodiments 172-174, wherein the agent that treats erectile dysfunction is sildenafil, tadafenil, and phosphodiesterase type 5 inhibitors.
176. A pharmaceutical composition comprising a dual N-type and L-type calcium channel blocker selective for the N-type calcium channel, a calcineurin inhibitor, and optionally a pharmaceutically acceptable excipient.
177. The composition of embodiment 176, wherein the dual N-type and L-type calcium channel blocker selective for the N-type calcium channel is selected from the group consisting of: cilnidipine, Z-160, CNV2197944, or pharmaceutically acceptable salts thereof.
178. The composition of any one of embodiments 176-177, wherein the dual N-type and L-type calcium channel blocker selective for the N-type calcium channel is cilnidipine or a pharmaceutically acceptable salt thereof.
179. The composition of any one of embodiments 176-178, wherein the calcineurin inhibitor is a cyclosporine.
180. The composition of any one of embodiments 176-179, further comprising a non-steroidal anti-inflammatory drug.
181. The composition of embodiment 180, wherein the non steroidal anti-inflammatory drug is aspirin.

### EXAMPLES

Example 1: Cell based assay activity of cilnidipine, gabapentin, amlodipine, tetrodotoxin (TTX), and bupivacaine.

### Scope

The aim of this assay was to record IC₅₀ values for 4 compounds (defined below) on the voltage-gated sodium channel subtype 1.7 (Nav 1.7) on both the closed and the inactivated state using the automated patch clamp platform QPATCH II^{™}.

### Background

The Nav 1.7 sodium channel is expressed in the dorsal root ganglion (DRG) and plays a role in propagating the action potential in these cells. Opening of the Nav 1.7 sodium channel depolarizes the membrane potential. Following this depolarization, the channel enters an inactivated state that prevents the channel from "firing" again too early. After the cells' membrane potential has returned to the resting potential, the channel is slowly released from the inactivated state into the closed (resting) state. A state dependent block of a sodium channel.

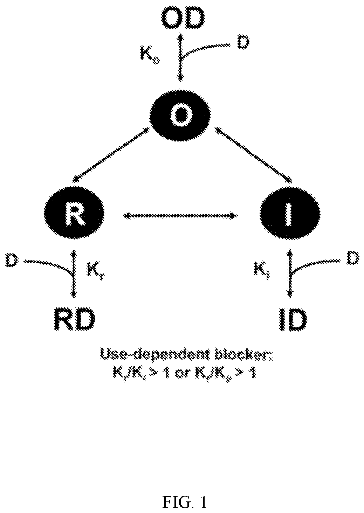

FIG. 1 is a schematic diagram based on the modulated hypothesis of drug binding to various states of a sodium channel (NaCh). *See* Benjamin et al. 2006 State-dependent compound inhibition of Nav 1.2 sodium channels using the FLIPR Vm dye: on-target and off-target effects of diverse pharmacological agents. J Biomol Screen. 2006 Feb; 11(1):29-39*.* The states represented are as follows: R (resting), O (open), I (inactivated). The model shows that a drug can directly interact with any of the 3 states. When a drug exhibits the same affinity for all 3 states, it is considered non-state dependent. When a drug exhibits greater affinity for 1 or 2 states of the channel than it does for the third, it is considered state-dependent. When a drug exhibits greater affinity for the open or inactivated states than it does for the resting state, it is considered use-dependent. A state-dependent blocker will act more potently in cells that fire a high frequency of action potentials. For this reason, drug development against voltage - gated sodium channels typically includes protocols that allow to distinguish between the modes of action.

### Materials and Methods:

### Compounds:

Cilnidipine, gabapentin, and amlodipine were obtained from SIGMA ALDRICH^{®}, tetrodotoxin (TTX) was obtained from ALOMONE LABS^{®}, and bupivacaine was obtained from AUROMEDICS^{®}. All the compounds were added to the external solution and a serial dilution of 6 concentrations were performed for each compound based on clinical Cₘₐₓ values.

### Cell line:

Chinese Hamster Ovary (CHO) cells stably expressing human Nav 1.7 were cultured according to the manufacturer's standard operating procedure (SOP) and enzymatically lifted into solution following Sophion's standard SOP.

### Solutions:

### External Solution: EC 0.0.0 NaCl-Ringer's Solution

| Chemical | Total Concentration |
|---|---|
| CaCl₂ (ID 2) | 2 mM |
| MgCl₂ (ID 15) | 1 mM |
| HEPES | 10 mM |
| KCL (ID 12) | 4 mM |
| NaCl (ID 17) | 145 mM |
| Glucose (ID 8) | 10 mM |

### Internal Solution: IC 7.0.0 KF-Ringer

| Chemical | Total Concentration |
|---|---|
| KF | 120 mM |
| KCL (ID 12) | 20 mM |
| HEPES | 10 mM |
| EGTA | 10 mM |
| pH: 7.2 mOsm with KOH: 300 mOsm | |

### Reference:

3 µM TTX in external solution

### Voltage Protocols:

Voltage protocol 1 (VP1): A voltage protocol designed to generate a current voltage relationship and to estimate the half inactivation potential (V1/2) was used. The first voltage protocol was used as quality control and the latter was used later in the experiment (see below).

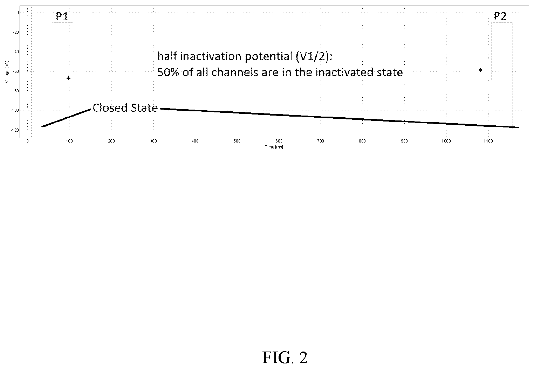

Voltage protocol 2 (VP2): Two pulse voltage protocol. FIG. 2 depicts a two pulse voltage protocol. The line marked with an asterisk indicates the voltage that was applied to the cell. In the beginning, all channels were in the closed state (V = -120 mV). A depolarization step to V = 10 mV (P1) activated all channels and produced the largest possible current.

Next, cells were kept at the V1/2 value for 1 s. Compounds that have an affinity for this state had the possibility to bind to this state during this time. A second stimulation to V = -10 mV activated all channels that were in the closed state (50%). Compound effects were assessed at both P1 and P2. State dependent compounds will show a different degree of inhibition at the two different pulses. This protocol was repeated every 10 s to monitor compound effects on the channel.

### Application Protocol:

### Liquid Periods

| Liquid | Volume (µl) | VP runs | Details |
|---|---|---|---|
| 1. Res: Saline | 5 | 12 | N/A |
| 2. Res: Saline | 5 | 1 | N/A |
| 3. Res: Saline | 0 | 6 | N/A |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 10 Res: Reference | 5 | 12 | N/A |

### Results:

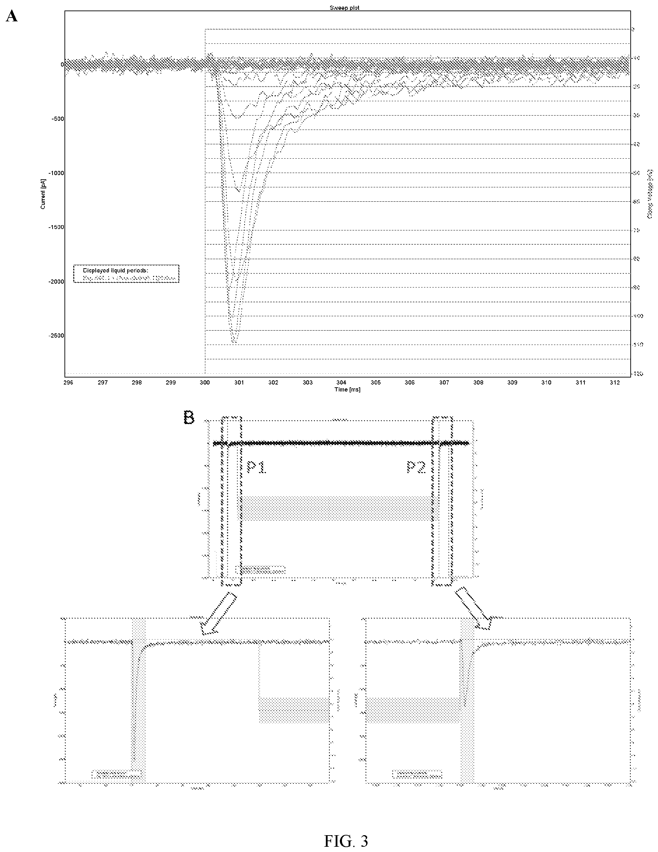

Raw traces can be seen in FIG. 3A and FIG. 3B. FIG. 3A is an overlay of inactivation current traces from VP1. FIG. 3B depicts current traces at closed and inactivated states from the two pulse voltage protocol (VP2). Current traces were expanded to indicate the current difference between the closed (P1) and inactivated (P2) states.

*Current over time plots for all compounds showing the decrease in current following compound exposure.*

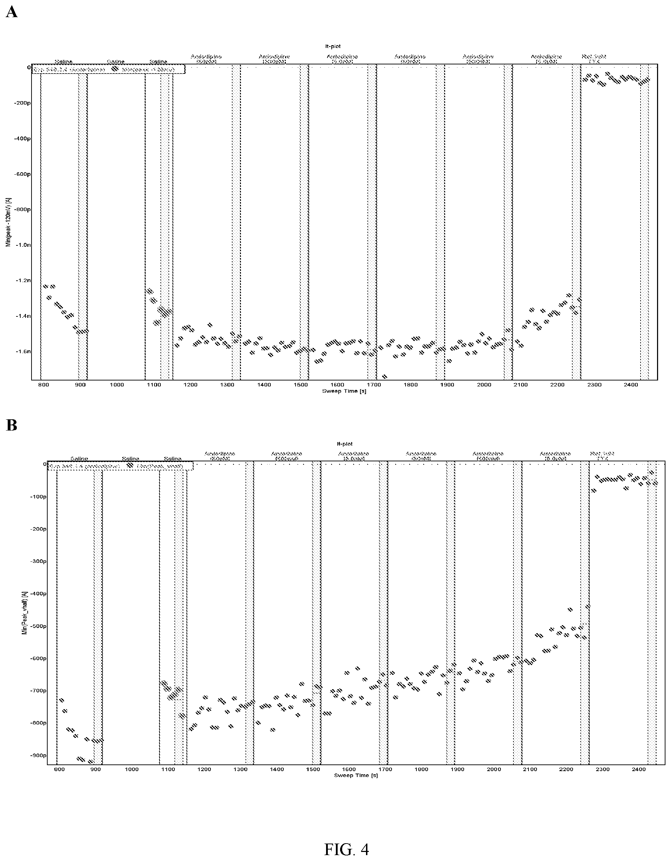

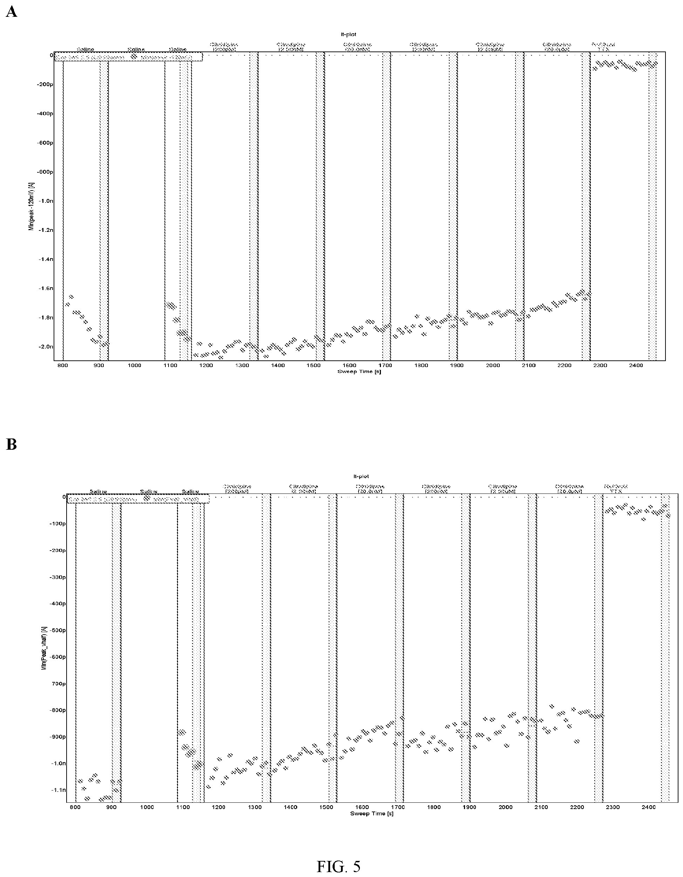

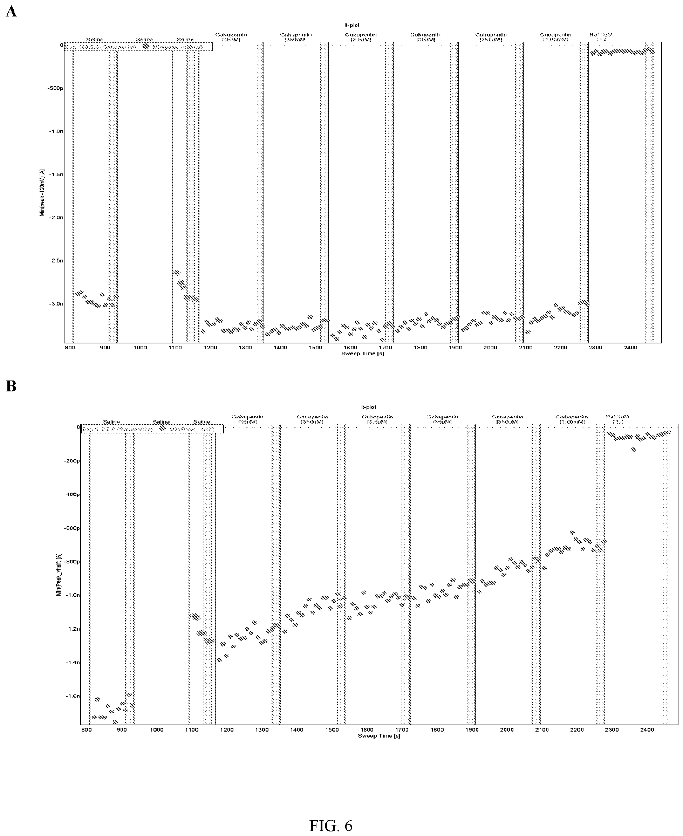

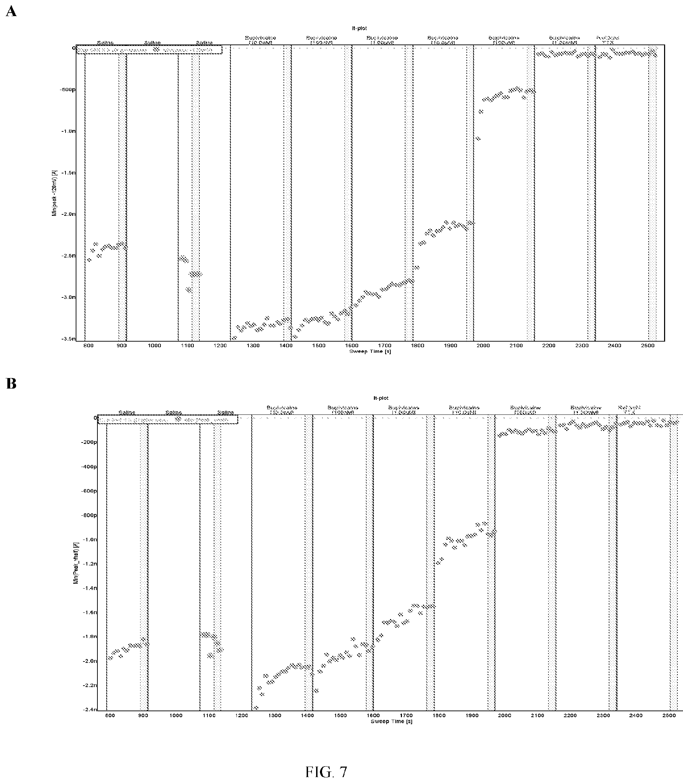

FIG. 4A depicts an It-plot for amlodipine at the closed state (P1). FIG. 4B depicts an It-plot for amlodipine at the inactivated state (P2). Concentrations of amlodipine were: 50pM, 500pM, 5nM, 50nM, 500nM, and 5µM. FIG. 5A depicts an It-plot for cilnidipine at the closed state (P1). FIG. 5B is an It-plot for cilnidipine at the inactivated state (P2). FIG. 6A depicts an It-plot for gabapentin at closed state (P1). FIG. 6B depicts an It-plot for gabapentin at inactivated state (P2). Concentrations of gabapentin were: 35nM, 350nM, 3.5µM, 35µM, 350µM, and 1mM.Concentrations of cilnidipine were: 200pM, 2nM, 20nM, 200nM, 2µM, and 20µM. FIG. 7A depicts an example of an It-plot for bupivacaine at closed state (P1). FIG. 7B depicts an It-plot for bupivacaine at inactivated state (P2). Concentrations of bupivacaine were: 10nM, 100nM, 1µM, 10µM, 100µM, and 1mM.

*Combined concentration response curve for the closed (P1) and the inactivated state (P2) for each compound.*

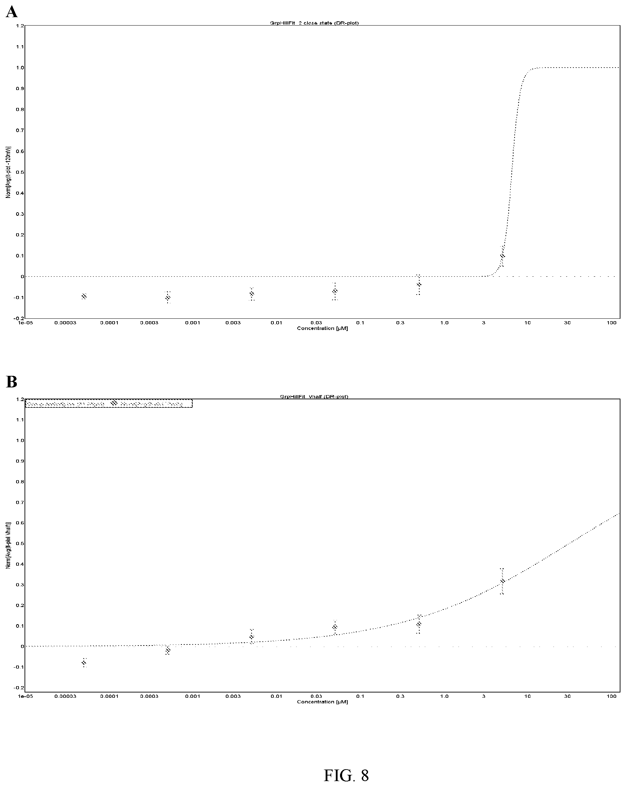

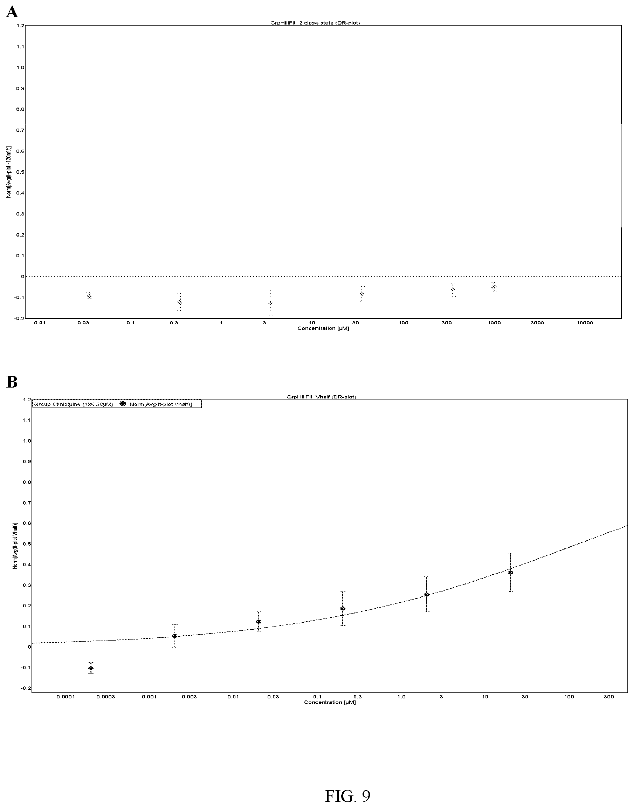

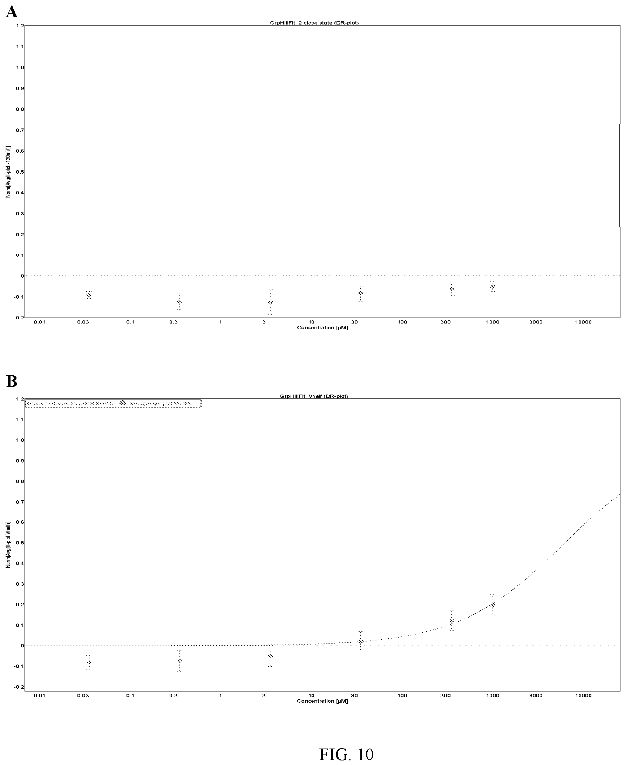

FIG. 8A depicts a group hill fit for amlodipine at closed state (P1) and FIG. 8B depicts a group hill fit for amlodipine at (P2) inactivated state. X-axis is the compound concentration and Y-axis is the inhibited rate with 1 set as maximum. FIG. 9A depicts a group hill fit for cilnidipine at closed state (P1) and FIG. 9B depicts a group hill fit for cilnidipine at (P2) inactivated state. The X-axis is the compound concentration and Y-axis is the inhibited rate with 1 set as maximum. FIG. 10A depicts a group hill fit for gabapentin at closed state (P1) and FIG. 10B depicts (P2) inactivated state. The X-axis is the compound concentration and the Y-axis is the inhibited rate with 1 set as maximum.

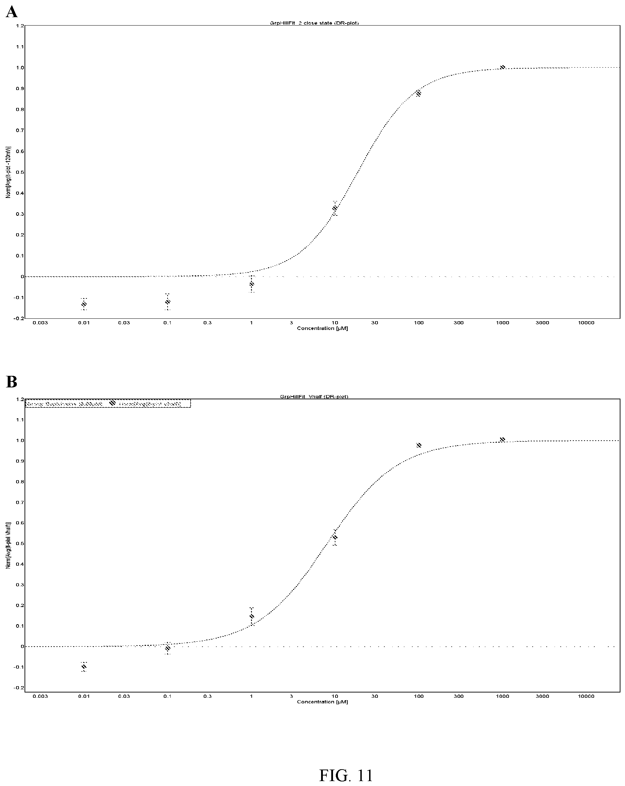

FIG. 11A depicts a group hill fit for bupivacaine at closed state (P1) and FIG. 11B depicts (P2) inactivated state. The X-axis is the compound concentration and the Y-axis is the inhibited rate with 1 set as maximum.

### Summarized relative Nav 1.7 channel inhibition

At the plasma concentrations achieved with a 10 mg dose of cilnidipine, versus a clinically comparable 5 mg dose of amlodipine, cilnidipine has a 9.5% higher relative inhibition of Nav 1.7 than amlodipine.

**Example 2:** Relative Nav 1.7 inhibition of the Nav 1.7 sodium channel of amlodipine, cilnidipine, nifedipine, gabapentin, bupivacaine, lidocaine, and DMSO.

### Materials and Methods:

The voltage protocol for this experiment are the same as described in connection with Example 1.

**Table 4. Testing concentration for each compound.**

| Compound | Oral dose of compound | Cmax^{†} | Cmax used in experiments | Concentrations tested |
|---|---|---|---|---|
| nifedipine | 40 mg | 36.55 ± 6.76 ng/ml | 40mg/ml (~1 15nM) | 0.1 µM |
| cilnidipine | 10 mg | 5.9 ± 1.2 ng/ml | 10ng/ml (~20nM) | 0.02 µM |
| cilnidipine | 20 mg | 37.0 | N/A | N/A |
| amlodipine | 10 mg | 8.43 ± 2.24 µg/ml | 2ng/ml (~4nM) | 0.005 µM |
| gabapentin | 1800 mg | N/A | 6µg/ml (~35 µM) | 35 µM |
| bupvicacaine HCl* | N/A | N/A | N/A | 1 µM |
| lidocaine HCl* | N/A | N/A | N/A | 10 µM |
| DMSO* | N/A | N/A | N/A | N/A |

| | | | | |
|---|---|---|---|---|
| * Denotes reference compound †Denotes reference value | | | | |

*Summary of inhibition rate for closed and inactivated state at*/*around Cmax concentrations.*

**Table 5. Experimental data of mean values of inhibition for closed and inactivated state of the Nav 1.7 sodium channel.**

| Compound | Mean % inhibition-closed state | Mean % inhibition-inactivated state | Std. error closed state | Std. error inhibition state |
|---|---|---|---|---|
| nifedipine | -2.2 | 37.5 | 2.0 | 3.7 |
| cilnidipine | -6.7 | 18.5 | 3.7 | 3.1 |
| amlodipine | -7.4 | 7.8 | 3.8 | 0.2 |
| gabapentin | -5.2 | 6.3 | 3.2 | 0.1 |
| bupivacaine | 2.5 | 20 | 2.4 | 3.6 |
| lidocaine | 40.9 | 89.0 | 3.3 | 2.1 |
| DMSO | -2.7 | 38.7 | 2.9 | 4.9 |

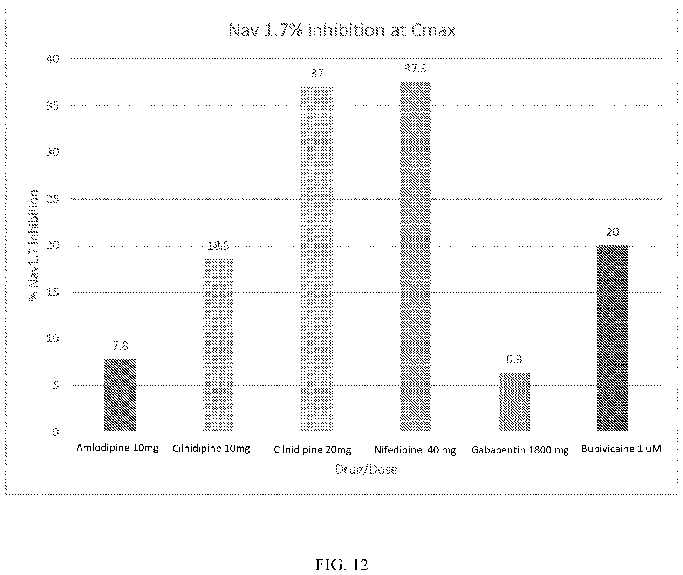

FIG. 12 depicts percent Nav 1.7 inactivated state sodium channel inhibition at Cₘₐₓ for nifedipine 40 mg, cilnidipine 10 mg, cilnidipine 20 mg, amlodipine 10 mg, gabapentin 1800 mg, and bupivacaine 1 µM.

### Results

Cilnidipine has clinically meaningful activity at the sodium channel subunit Nav 1.7, a target in the development of treatments for neuropathic pain. The activity of cilnidipine was about 5X that of amlodipine at comparable clinical doses (amlodipine 5 mg to cilnidipine 10 mg and amlodipine 10 mg to cilnidipine 20 mg). In addition, cilnidipine at 20 mg dose shows activity that is greater than gabapentin, another drug used to treat neuropathic pain. The activity of cilnidipine at a 20 mg dose appears to be greater than a sodium channel blocker and local anesthetic, bupivacaine, which is used sometimes to block postoperative pain with regional anesthesia or wound infiltration. The data shows, that the compounds in terms of efficacy would be ranked gabapentin < amlodipine < cilnidipine -nifedipine.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

The present disclosure will now be further defined in the following paragraphs:
1. A method of treating a disease or disorder associated with dysregulation of blood flow and sympathetic nervous system overactivity in a subject in need thereof, comprising administering a therapeutically effective amount of a dual N-type and L-type calcium channel blocker selective for the N-type calcium channel to the subject.
2. The method of paragraph 1, wherein one or more side effects experienced by the subject after administration of the dual N-type and L-type calcium channel blocker selective for the N-type calcium channel are less severe or less frequent than as compared to the side effects experienced by a subject after administration of a therapeutically effective amount of a non-N-selective calcium channel blocker useful to treat the disease or disorder.
3. The method of any one of paragraphs 1-2, wherein the disease or disorder associated with dysregulation of blood flow and sympathetic nervous system overactivity is characterized by neuropathic pain, vasoconstriction, dysesthetic pain, hyperesthetic pain, allodynia, lancinating pain, crampy pain, dull pain, burning pain, body temperature changes of the subject, changes in skin or tissue color, edema, changes in skin turgor, rubor, pallor, cyanosis, vasospasm, or any combination thereof.
4. The method of any one of paragraphs 1-3, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
5. The method of any one of paragraphs 1-4, wherein the disease or disorder is Raynaud's syndrome.
6. The method of any one of paragraphs 4-5, wherein Raynaud's syndrome is selected from the group consisting of primary Raynaud's syndrome; secondary Raynaud's syndrome; Raynaud's syndrome of the nipple, nose, ear, penis, tongue, and/or any alar circulatory region.
7. The method of paragraph 6, wherein the Raynaud's syndrome is secondary Raynaud's syndrome.
8. The method of any one of paragraphs 2-7, wherein the non-N-selective calcium channel blocker is selected from the group consisting of nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine, nitrendipine, and pharmaceutically acceptable salts thereof.
9. The method of any one of paragraphs 2-8, wherein the side effects are selected from the group consisting of: constipation, nausea, headache, fatigue, rash, edema, pulmonary edema, peripheral edema, heart rate changes, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, itching, and combinations thereof.
10. The method of any one of paragraphs 5-7, wherein the subject is also diagnosed with hypertension; and wherein after administration of the dual N-type and L-type calcium channel blocker selective for the N-type calcium channel to the subject, the blood pressure of the subject is reduced.
11. The method of paragraph 10, wherein the systolic blood pressure of the subject is reduced by greater than 10 mm Hg.
12. The method of paragraph 7, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof.
13. The method of any one of paragraphs 7 and 12, wherein the subject is being treated for scleroderma.
14. The method of any one of paragraphs 7 and 12, wherein the subject is being treated for scleroderma with interstitial lung disease.
15. The method of any one of paragraphs 13-14, further comprising administering an agent selected from the group consisting of a calcineurin inhibitor, cyclophosphamide, nintedanib, methotrexate, mycophenolate, a glucocorticoid, a non steroidal anti-inflammatory drug, D-penicillamine, a diuretic, omeprazole, bosentan, epoprostenol, enalapril, lisinopril, captopril, or any combination thereof.
16. The method of paragraph 15, further comprising administering nintedanib.
17. The method of paragraph 15, further comprising administering a calcineurin inhibitor, a non-steroidal anti-inflammatory drug, or both.
18. The method of paragraph 17, wherein the dual N-type and L-type calcium channel blocker selective for the N-type calcium channel, the calcineurin inhibitor, and the non-steroidal anti-inflammatory drug are administered separately, sequentially, or simultaneously.
19. The method of paragraph 18, wherein the dual N-type and L-type calcium channel blocker selective for the N-type calcium channel, the calcineurin inhibitor, and the non-steroidal anti-inflammatory drug are administered simultaneously.
20. The method of paragraph 19, wherein the dual N-type and L-type calcium channel blocker selective for the N-type calcium channel, the calcineurin inhibitor, and the non-steroidal anti-inflammatory drug are administered simultaneously as a fixed dosage form.
21. The method of any one of paragraphs 15 and 17-20, wherein the calcineurin inhibitor is a cyclosporine.
22. The method of any one of paragraphs 15 and 17-20, wherein the non steroidal anti-inflammatory drug is aspirin.
23. The method of any one of paragraphs 7 and 12, wherein the subject is being treated for lupus.
24. The method of paragraph 23, further comprising administering an agent selected from the group consisting of an antimalarial drug, a non-steroidal anti-inflammatory drug, belimumab, a corticosteroid, an immunosuppressant, or any combination thereof.
25. The method of any one of paragraphs 7 and 12, wherein the subject is being treated for rheumatoid arthritis.
26. The method of paragraph 25, further comprising administering an agent selected from the group consisting of methotrexate, sulfasalazine, a non-steroidal anti-inflammatory drug, a corticosteroid, a biologic, or any combination thereof.
27. The method of any one of paragraphs 7 and 12, wherein the subject is being treated for Sjögren's syndrome.
28. The method of paragraph 27, further comprising administering an agent selected from the group consisting of: plaquenil, an antimalarial drug, evoxac, cevimeline, infliximab, or any combination thereof.
29. The method of any one of paragraphs 7 and 12, wherein the subject is being treated for idiopathic pulmonary fibrosis.
30. The method of paragraph 29, further comprising administering an agent selected from the group consisting of: nintedanib, pirfenidone, or any combination thereof.
31. A method of treating a disease or disorder characterized by vasoconstriction or neuropathic pain in a subject in need thereof, the method comprising (a) determining that the disease or disorder is associated with vasoconstriction or neuropathic pain; and (b) administering to the subject a therapeutically effective amount of a dual N-type and L-type calcium channel blocker selective for the N-type calcium channel.
32. The method of paragraph 31, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
33. The method of any one of paragraphs 31-32, wherein the disease or disorder is Raynaud's syndrome.
34. The method of any one of paragraphs 32-33, wherein Raynaud's syndrome is selected from the group consisting of primary Raynaud's syndrome; secondary Raynaud's syndrome; Raynaud's syndrome of the nipple, nose, ear, penis, tongue, and/or any alar circulatory region.
35. The method of paragraph 34, wherein the Raynaud's syndrome is secondary Raynaud's syndrome.
36. The method of any one of paragraphs 33-35, wherein the subject is also diagnosed with hypertension; and wherein after administration of the dual N-type and L-type calcium channel blocker selective for the N-type calcium channel to the subject, the blood pressure of the subject is reduced.
37. The method of paragraph 36, wherein the systolic blood pressure of the subject is reduced by greater than 10 Hg.
38. The method of paragraph 35, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof.
39. The method of paragraph 38, wherein the subject is being treated for scleroderma.
40. The method of paragraph 38, wherein the subject is being treated for scleroderma with interstitial lung disease.
41. The method of any one of paragraphs 39-40, further comprising administering an agent selected from the group consisting of a calcineurin inhibitor, cyclophosphamide, nintedanib, methotrexate, mycophenolate, a glucocorticoid, a non steroidal anti-inflammatory drug, D-penicillamine, a diuretic, omeprazole, bosentan, epoprostenol, enalapril, Lisinopril, captopril, or any combination thereof.
42. The method of any one of paragraphs 39-41, further comprising administering nintedanib.
43. The method of any one of paragraphs 41-42, further comprising administering a calcineurin inhibitor, a non-steroidal anti-inflammatory drug, or both.
44. The method of paragraph 43, wherein the dual N-type and L-type calcium channel blocker selective for the N-type calcium channel, the calcineurin inhibitor, and the non-steroidal anti-inflammatory drug are administered separately, sequentially, or simultaneously.
45. The method of paragraph 44, wherein the dual N-type and L-type calcium channel blocker selective for the N-type calcium channel, the calcineurin inhibitor, and the non-steroidal anti-inflammatory drug are administered simultaneously.
46. The method of paragraph 45, wherein the dual N-type and L-type calcium channel blocker selective for the N-type calcium channel, the calcineurin inhibitor, and the non-steroidal anti-inflammatory drug are administered simultaneously as a fixed dosage form.
47. The method of any one of paragraphs 41 and 43-46, wherein the calcineurin inhibitor is a cyclosporine.
48. The method of any one of paragraphs 41 and 43-47, wherein the non steroidal anti-inflammatory drug is aspirin.
49. The method of paragraph 38, wherein the subject is being treated for lupus.
50. The method of paragraph 49, further comprising administering an agent selected from the group consisting of an antimalarial drug, a non-steroidal anti-inflammatory drug, belimumab, a corticosteroid, an immunosuppressant, or any combination thereof.
51. The method of paragraph 38, wherein the subject is being treated for rheumatoid arthritis.
52. The method of paragraph 51, further comprising administering an agent selected from the group consisting of methotrexate, sulfasalazine, a non-steroidal anti-inflammatory drug, a corticosteroid, a biologic, or any combination thereof.
53. The method of paragraph 38, wherein the subject is being treated for Sjögren's syndrome.
54. The method of paragraph 53, further comprising administering an agent selected from the group consisting of: plaquenil, an antimalarial drug, evoxac, cevimeline, infliximab, or any combination thereof.
55. The method of paragraph 38, wherein the subject is being treated for idiopathic pulmonary fibrosis.
56. The method of paragraph 55, further comprising administering an agent selected from the group consisting of: nintedanib, pirfenidone, or any combination thereof.
57. The method of any one of paragraphs 31-56, comprising administering at least one additional calcium channel blocker to the subject.
58. The method of paragraph 57, wherein the at least one additional calcium channel blocker is selected from the group consisting of amlodipine, nifedipine, nicardipine, nimodipine, verapamil, diltiazem, felodipine, isradipine, nisoldipine, and nitrendipine.
59. A method of reducing a sensation of burning pain, paresthesia, dysesthesia, hypoesthesia, allodynia, or hyperesthesia in a subject in need thereof, comprising administering a therapeutically effective amount of a dual N-type and L-type calcium channel blocker selective for the N-type calcium channel to a subject.
60. The method of any one of paragraphs 1-59, further comprising administering to the subject a therapeutically effective amount of an agent that increases blood pressure.
61. The method of paragraph 60, wherein the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.
62. The method of paragraph 61, wherein the blood pressure of the subject before and after administration of the dual N-type and L-type calcium channel blocker selective for the N-type calcium channel and the agent that increases blood pressure is substantially the same.
63. The method of any one of paragraphs 1-62, wherein the bone density of the subject does not decrease.
64. The method of any one of paragraphs 1-62, further comprising selecting a subject identified or diagnosed as having reduced bone density for the treatment.
65. The method of paragraph 64, wherein the subject identified or diagnosed as having reduced bone density is afflicted with osteoporosis.
66. The method of any one of paragraphs 64-65, wherein the subject is female.
67. The method of any one of paragraphs 1-66, further comprising selecting a subject identified or diagnosed as having reduced renal function for the treatment.
68. The method of paragraph 67, wherein the renal function of the patient is not reduced after treatment.
69. A method of reducing pain or discomfort in a subject in need thereof caused by a reduction of body temperature in the subject, comprising administering an effective amount of a dual N-type and L-type calcium channel blocker selective for the N-type calcium channel to the subject, wherein the reduction of body temperature in the subject is caused by an exposure of the subject to air having a temperature of less than 25°C.
70. The method of paragraph 69, wherein vasoconstriction in the subject is reduced after administering the dual N-type and L-type calcium channel blocker selective for the N-type calcium channel.
71. The method of any one of paragraphs 69-70, wherein the reduction of body temperature in the subject is caused by an exposure of the subject to air having a temperature of less than 10°C.
72. The method of any one of paragraphs 69-71, wherein the reduction of body temperature in the subject comprises reduction in the temperature of a digit, an extremity, or alar circulatory region of the subject.
73. The method of any one of paragraphs 69-72, wherein the reduction of body temperature in the subject comprises reduction in the temperature of a hand or a foot of the subject.
74. A method of reducing susceptibility of a subject to cold-induced pain or discomfort, comprising: administering an effective amount of a dual N-type and L-type calcium channel blocker selective for the N-type calcium channel to the subject.
75. A method of treating cold-induced pain or discomfort in a subject, comprising: administering an effective amount of a dual N-type and L-type calcium channel blocker selective for the N-type calcium channel to the subject.
76. The method of any one of paragraphs 74-75, wherein the subject experiences a lesser degree of the pain or discomfort upon exposure to air having a temperature of less than 25 °C than as compared to a subject that is not administered a dual N-type and L-type calcium channel blocker selective for the N-type calcium channel and is exposed to air having a temperature of less than 25 °C.
77. The method of any one of paragraphs 1-76, wherein the dual N-type and L-type calcium channel blocker selective for the N-type calcium channel is selected from the group consisting of: cilnidipine, Z-160, CNV2197944, or pharmaceutically acceptable salts thereof.
78. The method of any one of paragraphs 1-76, wherein the dual N-type and L-type calcium channel blocker selective for the N-type calcium channel is cilnidipine or a pharmaceutically acceptable salt thereof.
79. The method of any one of paragraphs 1-78, wherein the dosage of the dual N-type and L-type calcium channel blocker selective for the N-type calcium channel is about 0.005 mg/kg to about 2 mg/kg.
80. The method of any one of paragraphs 1-78, wherein the dosage of the dual N-type and L-type calcium channel blocker selective for the N-type calcium channel is about 0.06 mg/kg to about 0.3 mg/kg.
81. The method of any one of paragraphs 1-78, wherein the dosage of the dual N-type and L-type calcium channel blocker selective for the N-type calcium channel is about 0.1 mg/kg to about 0.18 mg/kg.
82. The method of any one of paragraphs 1-81, wherein the dual N-type and L-type calcium channel blocker selective for the N-type calcium channel is administered orally, parenterally, transdermally, by inhalation, intranasally, sublingually, neuraxially, or ocularly.
83. The method of any one of paragraphs 1-82, wherein each administration of the dual N-type and L-type calcium channel blocker selective for the N-type calcium channel is separated by at least about 24 hours.
84. The method of any one of paragraphs 1-82, wherein each administration of the dual N-type and L-type calcium channel blocker selective for the N-type calcium channel is separated by at least about 48 hours.
85. The method of any one of paragraphs 1-82, wherein each administration of the dual N-type and L-type calcium channel blocker selective for the N-type calcium channel is separated by at least about 72 hours.
86. The method of any one of paragraphs 1-82, wherein each administration of the dual N-type and L-type calcium channel blocker selective for the N-type calcium channel is separated by at least about 1 week.
87. The method of any one of paragraphs 1-86, wherein the dual N-type and L-type calcium channel blocker selective for the N-type calcium channel causes sympathetic tone diminution, direct smooth muscle relaxation, or both in the subject.
88. The method of any one of paragraphs 1-87, wherein the dual N-type and L-type calcium channel blocker selective for the N-type calcium channel exhibits at least a 50-fold selectivity for the N-type calcium channel over an L-type calcium channel.
89. The method of any one of paragraphs 1-87, wherein the dual N-type and L-type calcium channel blocker selective for the N-type calcium channel exhibits a 50-fold to 100-fold selectivity for the N-type calcium channel over an L-type calcium channel.
90. The method of any one of paragraphs 1-89, wherein the dual N-type and L-type calcium channel blocker selective for the N-type calcium channel further inhibits a Nav 1.7 sodium channel.
91. The method of any one of paragraphs 1-90, wherein after administration of the dual N-type and L-type calcium channel blocker selective for the N-type calcium channel nitric oxide is not increased in the subject.
92. The method of any one of paragraphs 1-91, wherein after administration of the dual N-type and L-type calcium channel blocker selective for the N-type calcium channel endothelial dysfunction in the subject is improved.
93. The method of any one of paragraphs 1-92, wherein after administration of the dual N-type and L-type calcium channel blocker selective for the N-type calcium channel oxidative stress in the subject is decreased.
94. The method of any one of paragraphs 1-93, wherein an antioxidant is not administered to the subject.
95. The method of paragraph 94, wherein the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.
96. A method of treating a disease or disorder associated with dysregulation of blood flow and sympathetic nervous system overactivity in a subject in need thereof, comprising administering a therapeutically effective amount of a Nav 1.7 sodium channel blocker to the subject.
97. The method of paragraph 96, wherein the disease or disorder associated with dysregulation of blood flow and sympathetic nervous system overactivity is characterized by neuropathic pain, vasoconstriction, dysesthetic pain, hyperesthetic pain, allodynia, lancinating pain, crampy pain, dull pain, burning pain, body temperature changes of the subject, changes in skin or tissue color, edema, changes in skin turgor, rubor, pallor, cyanosis, vasospasm, or any combination thereof.
98. The method of any one of paragraphs 96-97, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia,or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.
99. The method of any one of paragraphs 96-98, wherein the disease or disorder is Raynaud's syndrome.
100. The method of paragraph 99, wherein Raynaud's syndrome is selected from the group consisting of: primary Raynaud's syndrome; secondary Raynaud's syndrome; Raynaud's syndrome of the nipple, nose, ear, penis, tongue, and/or any alar circulatory region.
101. The method of any one of paragraphs 96-100, wherein the Nav 1.7 sodium channel blocker is cilnidipine or a pharmaceutically acceptable salt thereof.
102. The method of any one of paragraphs 96-101, wherein the dosage of the Nav 1.7 sodium channel blocker is about 0.005 mg/kg to about 2 mg/kg.
103. The method of any one of paragraphs 96-101, wherein the dosage of the Nav 1.7 sodium channel blocker is about 0.06 mg/kg to about 0.3 mg/kg.
104. The method of any one of paragraphs 96-101, wherein the dosage of the Nav 1.7 sodium channel blocker is about 0.1 mg/kg to about 0.18 mg/kg.
105. The method of any one of paragraphs 96-104, further comprising administering a therapeutically effective amount of an agent that increases blood pressure to the subject.
106. The method of paragraph 105, wherein the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.
107. The method of any one of paragraphs 105-106, wherein the blood pressure of the subject before and after administration of the Nav 1.7 sodium channel blocker and the agent that increases blood pressure is substantially the same.
108. The method of any one of paragraphs 96-107, wherein the Nav 1.7 sodium channel blocker is administered orally, parenterally, transdermally, by inhalation, intranasally, sublingually, neuraxially, or ocularly.
109. The method of any one of paragraphs 96-108, wherein the bone density of the subject does not decrease.
110. The method of any one of paragraphs 96-109, further comprising selecting a subject identified or diagnosed as having reduced bone density for the treatment.
111. The method of paragraph 110, wherein the subject identified or diagnosed as having reduced bone density is afflicted with osteoporosis.
112. The method of paragraph 111, wherein the subject is female.
113. The method of any one of paragraphs 96-112, wherein the health or functioning of a kidney in the subject is improved.
114. The method of any one of paragraphs 96-113, further comprising selecting a subject identified or diagnosed as having reduced renal function for the treatment.
115. The method of paragraph 114, wherein the renal function of the patient is not reduced after treatment.
116. The method of paragraph 115, wherein the renal function of the patient is improved after treatment.
117. The method of any one of paragraphs 96-116, wherein each administration of the Nav 1.7 sodium channel blocker is separated by at least about 24 hours.
118. The method of any one of paragraphs 96-116, wherein each administration of the Nav 1.7 sodium channel blocker is separated by at least about 48 hours.
119. The method of any one of paragraphs 96-116, wherein each administration of the Nav 1.7 sodium channel blocker is separated by at least about 72 hours.
120. The method of any one of paragraphs 96-116, wherein each administration of the Nav 1.7 sodium channel blocker is separated by at least about 1 week.
121. The method of any one of paragraphs 96-120, wherein the Nav 1.7 sodium channel blocker causes sympathetic tone diminution, direct smooth muscle relaxation, or both in the subject.
122. The method of any one of paragraphs 96-121, wherein after administration of the Nav 1.7 sodium channel blocker nitric oxide is not increased in the subject.
123. The method of any one of paragraphs 96-122, wherein an antioxidant is not administered to the subject.
124. The method of paragraph 123, wherein the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.
125. A pharmaceutical composition comprising a dual N-type and L-type calcium channel blocker selective for the N-type calcium channel, an agent that increases blood pressure, and optionally a pharmaceutically acceptable excipient.
126. The composition of paragraph 125, wherein the dual N-type and L-type calcium channel blocker selective for the N-type calcium channel is cilnidipine or a pharmaceutically acceptable salt thereof.
127. The composition of any one of paragraphs 125-126, wherein the agent that increases blood pressure is selected from the group consisting of: midodrine, cortisone, prednisone, trimipramine, and venlafaxine.

## Claims

1. A compound which is cilnidipine, or a pharmaceutically acceptable salt thereof, for use in treating a disease or disorder **characterized by** vasoconstriction or neuropathic pain in a subject in need thereof, the use comprising (a) determining that the disease or disorder is associated with vasoconstriction or neuropathic pain; and (b) administering to the subject a therapeutically effective amount of cilnidipine.

2. The compound for use of claim 1, wherein the disease or disorder is selected from the group consisting of: Raynaud's syndrome (e.g., primary Raynaud's syndrome or secondary Raynaud's syndrome); scleroderma or systemic sclerosis; complex regional pain syndrome Type I; complex regional pain syndrome Type II; nerve pain after surgery; burning pain during or after nerve compression; perception of temperature changes during or after nerve compression; pain during or after a burn; burning dysesthesias; neuropathic pain; erythromelalgia; vascular mediated pain syndromes; spinal stenosis; lumbar radiculopathy; failed back syndrome; cervical radiculopathy; causalgia; sympathetically mediated pain syndromes; trigeminal neuralgia; post-herpetic neuralgia; causalgia; fibromyalgia; diabetic neuropathy; chemotherapy induced neuropathy; restless legs syndrome; hot flashes; atherosclerosis, kidney disease or dysfunction, post-operative renal dysfunction, arthritis-related pain (e.g., osteoarthritis-related pain), drug related neuropathic pain, diseases of endothelial dysfunction, cardiac left ventricular disease or dysfunction; limb, extremity, surgical flap, post-surgical ischemia, or acute limb or extremity ischemia as a consequence of vasospasm or a thrombotic event; osteoporosis; heart remodeling after atrial fibrillation, QT prolongation in patients at risk for cardiovascular disease including hemodialysis patients; postoperative pain; hypertension; or treatment-resistant hypertension wherein other antihypertensive medications including but not limited to ace inhibitors, angiotensin receptor blockade agents, beta blockers, diuretics, alpha blockers, and other calcium channel blockers have dose limitations due to efficacy limitations or side effect occurrence.

3. The compound for use of any one of claims 1-2, wherein the disease or disorder is Raynaud's syndrome.

4. The compound for use of any one of claims 2-3, wherein Raynaud's syndrome is selected from the group consisting of: primary Raynaud's syndrome; secondary Raynaud's syndrome; Raynaud's syndrome of the nipple, nose, ear, penis, tongue, and/or any alar circulatory region.

5. The compound for use of claim 4, wherein the Raynaud's syndrome is secondary Raynaud's syndrome.

6. The compound for use of any one of claims 3-5, wherein the subject is also diagnosed with hypertension; and wherein after administration of the cilnidipine to the subject, the blood pressure of the subject is reduced.

7. The compound for use of claim 6, wherein the systolic blood pressure of the subject is reduced by greater than 10 Hg.

8. The compound for use of claim 5, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof.

9. The compound for use of claim 8, wherein the subject is being treated for scleroderma.

10. The compound for use of claim 8, wherein the subject is being treated for scleroderma with interstitial lung disease.

11. The compound for use of any one of claims 9-10, wherein the use further comprises administering an agent selected from the group consisting of: a calcineurin inhibitor, cyclophosphamide, nintedanib, methotrexate, mycophenolate, a glucocorticoid, a non-steroidal anti-inflammatory drug, D-penicillamine, a diuretic, omeprazole, bosentan, epoprostenol, enalapril, lisinopril, captopril, or any combination thereof.

12. The compound for use of any one of claims 9-11, wherein the use further comprises administering nintedanib.

13. The compound for use of any one of claims 11-12, wherein the use further comprises administering a calcineurin inhibitor, a non-steroidal anti-inflammatory drug, or both.

14. The compound for use of claim 13, wherein the cilnidipine, the calcineurin inhibitor, and the non-steroidal anti-inflammatory drug are administered separately, sequentially, or simultaneously.

15. The compound for use of claim 14, wherein the cilnidipine, the calcineurin inhibitor, and the non-steroidal anti-inflammatory drug are administered simultaneously.
